(19) Europäisches Patentamt
European Patent Office
Office européen des brevets

(11) EP 3 130 340 A1

(12) EUROPEAN PATENT APPLICATION
published in accordance with Art. 153(4) EPC

(43) Date of publication:
15.02.2017 Bulletin 2017/07

(21) Application number: 15776836.7

(22) Date of filing: 10.04.2015

(51) Int Cl.:
*A61K 31/7032* (2006.01) *A61K 31/7028* (2006.01)
*A61P 21/00* (2006.01) *A61P 25/28* (2006.01)

(86) International application number:
PCT/CN2015/076381

(87) International publication number:
WO 2015/154721 (15.10.2015 Gazette 2015/41)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA

(30) Priority: 10.04.2014 US 201461977637 P

(71) Applicant: Sinphar Tian-li Pharmaceutical Co., Ltd.
(Hangzhou)
Yuhang Economic Development Zone
Hangzhou
Zhejiang 311100 (CN)

(72) Inventors:
- SU, Muh-Hwan
Taipei (TW)
- LIN, Hang-Ching
Taipei (TW)
- HU, Ming-Kuan
Taipei (TW)
- LEE, Yita
Taipei (TW)
- WANG, Zhao-Ri
Taipei (TW)

(74) Representative: Boult Wade Tennant
Verulam Gardens
70 Gray's Inn Road
London WC1X 8BT (GB)

# (54) ISOACTEOSIDE DERIVATIVE AND PREPARATION METHOD AND USE THEREOF

(57) An isoacteoside derivative and forming method and uses thereof; the isoacteoside derivative has a structure of formula (I):

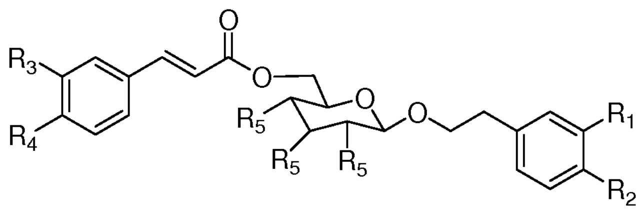
(I),

$R_1$ and $R_2$ are independently selected from hydrogen, halogen, hydroxyl and alkoxy oxyl; $R_3$ and $R_4$ are independently selected from hydroxyl, alkoxy oxyl and acyloxy; $R_5$ is independently selected from hydroxyl or acyloxy. Drugs comprsing the isoacteoside derivative have uses of treating or preventing amyloid peptide related diseases and preventing eye lesions and the like.

EP 3 130 340 A1

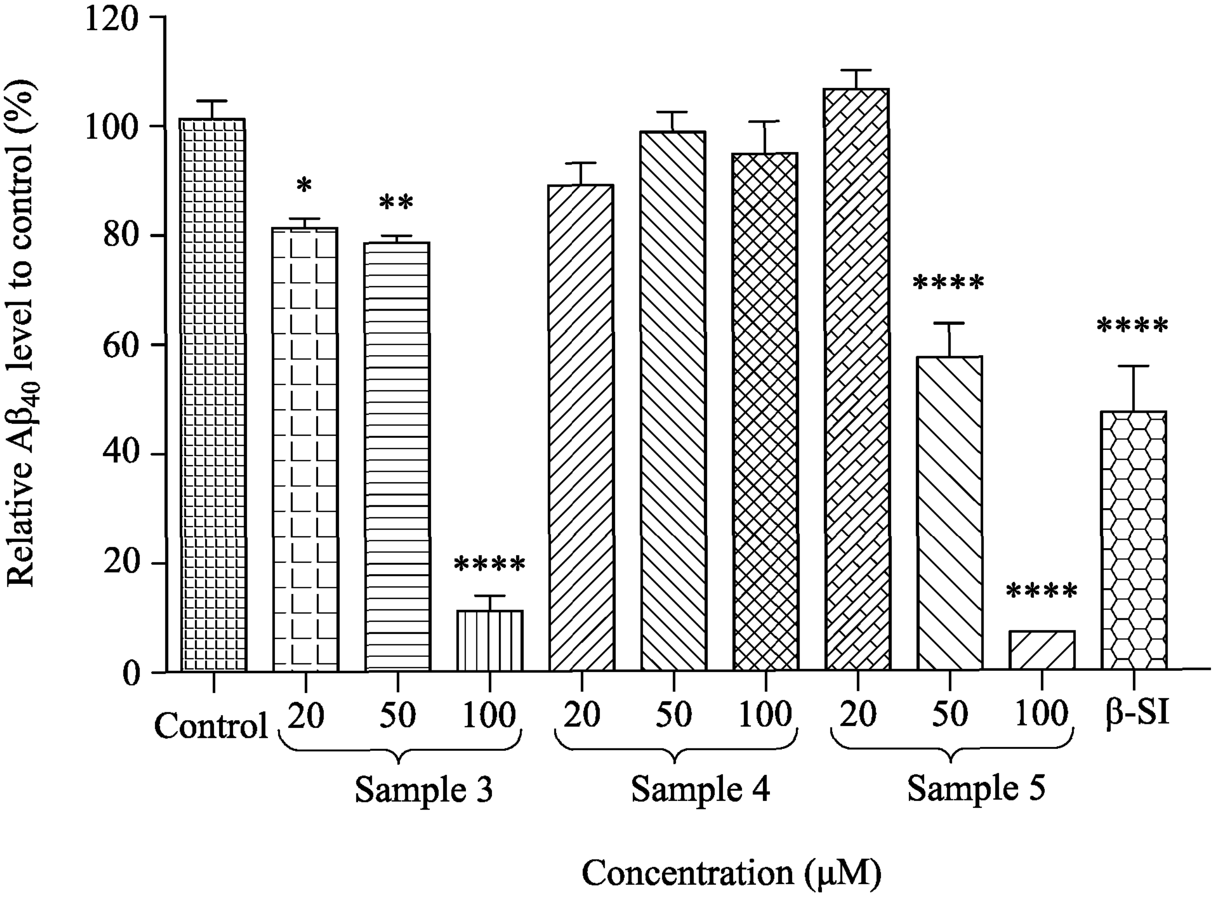
120
100
80
60
40
20
0
Relative Aβ40 level to control (%)
*
**
****
****
****
****
Control
20
50
100
20
50
100
20
50
100
β-SI
Sample 3
Sample 4
Sample 5
Concentration (μM)

Fig. 2A

## Description

Field of Invention

The present invention relates to an isoacteoside derivative and forming method and use thereof. More particularly, the present invention relates to an isoacteoside derivative, forming method thereof and use of medicine including the isoacteoside derivative.

Description of Related Art

Isoacteoside belongs to a kind of phenylpropanoid glycosides, and presents in many plants. For example, Cistanche also contains this ingredient. The structure of the isoacteoside includes dihydroxy-phenethyl-D-glucoside, cinnamic acid ester, and a monosaccharide.

Based on current research, the isoacteoside has efficacy of neuroprotection, liver protection, antioxidant, and reducing biological activity of amyloid peptide aggregation. According to the test results for researching activity of WO 2011 /157059 A1, when caffeoyl group was at the sixth position (such as isoacteoside), the activity of inhibiting β-amyloid peptides (Aβ) accumulation was better, and when the caffeoyl group was at the fourth position (such as acteoside), the activity decreased. These results show that the position of the caffeoyl group has a great effect on the activity of the isoacteoside.

Further, in pharmacological mechanism of isoacteoside for reducing Aβ aggregation, one possibility is that the bisphenol group of catechol and transition metals (such as copper, iron, zinc, etc) have a metal chelation reaction. A phenylethanoid group at the first position also includes a catechol group. Therefore, the phenylethanoid group should have a similar effect of metal chelation, and may be a necessary active group.

Accordingly, the present invention synthesizes a series of isoacteoside derivatives, which have efficacy of treating of preventing amyloid-related diseases (such as neuroprotection, reducing amyloid peptide aggregation, neurodegenerative disease, and eye disease).

## SUMMARY

An aspect of the present invention provides an isoacteoside derivative, having a structure of formula (I):

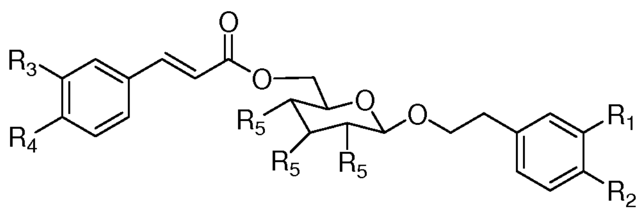

(I),

in formula (I), $R_1$ and $R_2$ being independently selected from hydrogen, halogen, a hydroxy group, or a hydrocarboxyl group, $R_3$ and $R_4$ being independently selected from a hydroxy group, a hydrocarboxyl group, or an acyloxy group, and $R_5$ being independently selected from a hydroxy group or an acyloxy group.

According to one embodiment of the present invention, when at least one of $R_1$ and $R_2$ is the hydrocarboxyl group, the at least one of $R_1$ and $R_2$ is independently selected from an alkoxy group, an alkenyloxy group, or an aryloxy group.

According to one embodiment of the present invention, when at least one of $R_1$ and $R_2$ is the alkoxy group, the at least one of $R_1$ and $R_2$ is a methoxy group.

According to one embodiment of the present invention, when at least one of $R_1$ and $R_2$ is the alkenyloxy group, the at least one of $R_1$ and $R_2$ is an allyloxy group.

According to one embodiment of the present invention, when at least one of $R_1$ and $R_2$ is the aryloxy group, the at least one of $R_1$ and $R_2$ is a benzyloxy group.

According to one embodiment of the present invention, when at least one of $R_3$ and $R_4$ is the hydrocarboxyl group, the at least one of $R_3$ and $R_4$ is independently selected from an alkenyloxy group or an aryloxy group.

According to one embodiment of the present invention, when at least one of $R_3$ and $R_4$ is the alkenyloxy group, the at least one of $R_3$ and $R_4$ is an allyloxy group.

According to one embodiment of the present invention, when at least one of $R_3$ and $R_4$ is the aryloxy group, the at least one of $R_3$ and $R_4$ is a benzyloxy group.

According to one embodiment of the present invention, when at least one of $R_3$ and $R_4$ is the acyloxy group, the at least one of $R_3$ and $R_4$ is an acetoxy group.

According to one embodiment of the present invention, $R_3$ and $R_4$ are the same substituent.

According to one embodiment of the present invention, when $R_5$ is the acyloxy group, $R_5$ is an acetoxy group.

According to one embodiment of the present invention, $R_5$ are the same substituent.

According to one embodiment of the present invention, the isoacteoside derivative is selected from following structures:

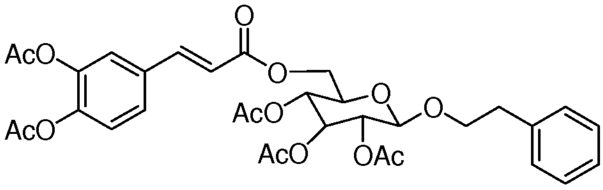
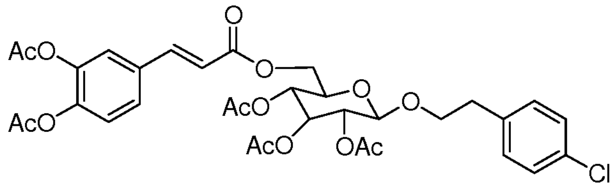

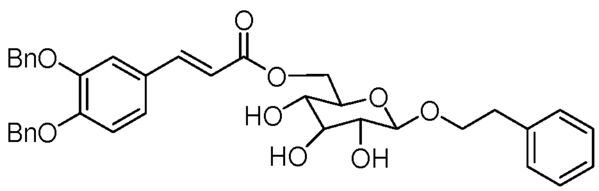
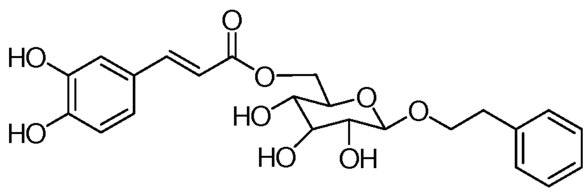

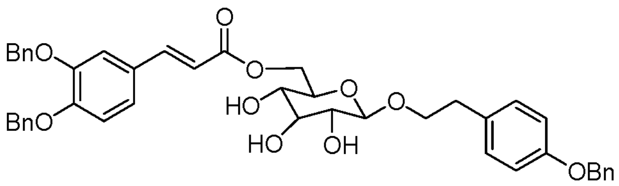
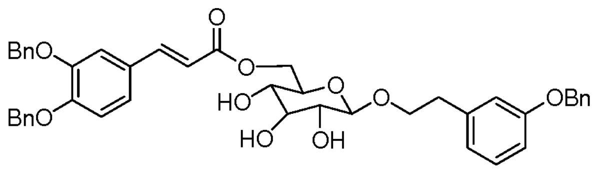

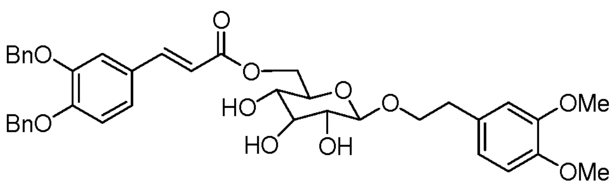
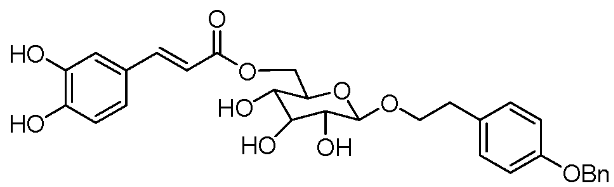

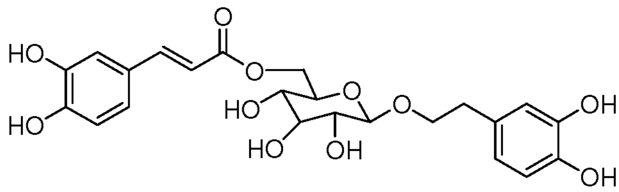

Another aspect of the present invention provides a use of a medicine for preventing or treating an amyloid-related disease, which the medicine includes the aforementioned isoacteoside derivative.

Preferably, the amyloid-related disease is a neurodegenerative disease.

Preferably, the amyloid-related disease is Alzheimer's disease, mild cognitive impairment, Lewy body dementia, Down's syndrome, hereditary cerebral hemorrhage with amyloidosis-Dutch type, the Guam Parkinson-Dementia complex, progressive supranuclear palsy, multiple sclerosis, Creutzfeld Jacob disease, Parkinson's disease, frontotemporal dementia, Pick's disease, amyotrophic lateral sclerosis, inclusion-body myositis, adult-onset diabetes, senile cardiac amyloidosis, or endocrine tumor.

According to one embodiment of the present invention, the amyloid is β-amyloid peptide.

Yet another aspect of the present invention provides a use of a medicine for preventing an eye disease, which the medicine includes the aforementioned isoacteoside derivative.

Preferably, the eye disease is neuronal degeneration, visual cortical defect, glaucoma, cataract, ocular amyloidosis, macular degeneration, optic nerve drusen, optic neuropathy, optic neuritis, or lattice corneal dystrophy.

Yet another aspect of the present invention provides a method for forming an isoacteoside derivative, including reacting a compound having a structure of formula (II) with β-D-glucose pentaacetate to form a compound having a structure of formula (III), which formula (II) is:

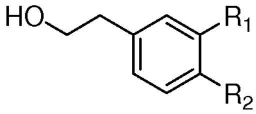 (II),

and formula (II) is:

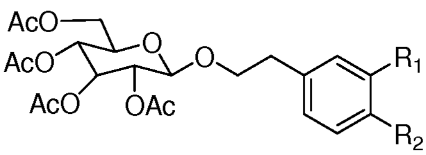 (III).

In formula (II) and formula (III), $R_1$ and $R_2$ being independently selected from hydrogen, chloride, or a methoxy group. Next, (1) the compound having the structure of formula (III) is reacted with a mixture of palladium on carbon and methanol, after removing the palladium on carbon and purifying, is mixed with potassium carbonate, allyl bromide, and acetone, and after refluxing, is stirred in a potassium hydroxide-methanol solution to form a compound having a structure of formula (IV-1), which formula (IV-1) is:

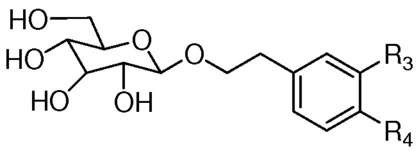 (IV-1),

in formula (IV-1), $R_3$ and $R_4$ being independently selected from hydrogen or an allyloxy group, (2) the compound having the structure of formula (III) is dissolved in methanol and mixed with sodium methoxide to form the compound having the structure of formula (IV-1), which $R_3$ and $R_4$ are independently selected from hydrogen, chloride, a methoxy group, or a benzyloxy group, or (3) the compound having the structure of formula (III) is reacted with acetyl chloride and methanol-dichloromethane to form a compound having a structure of formula (IV-2), which formula (IV-2) is:

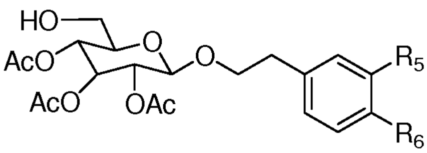 (IV-2),

in formula (IV-2), $R_5$ and $R_6$ being independently selected from hydrogen or chloride. Then, the compound having the structure of formula (IV-1) or the compound having the structure of formula (IV-2) is reacted with di-O-acetylferulic acid chloride, di-O-allylferulic acid chloride, or di-O-benzylferulic acid chloride in a solution of dichloromethane and pyridine to form a compound having a structure of any one of formulas (V-1)~(V-4), which formula (V-1) is:

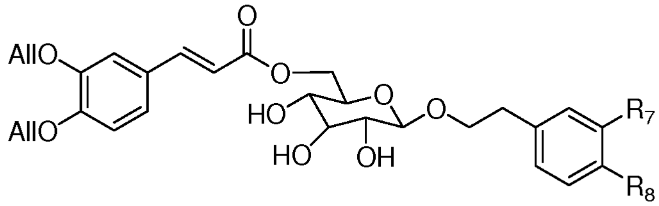

(V-1),

in formula (V-1), $R_7$ and $R_8$ being independently selected from hydrogen or an allyloxy group, formula (V-2) is:

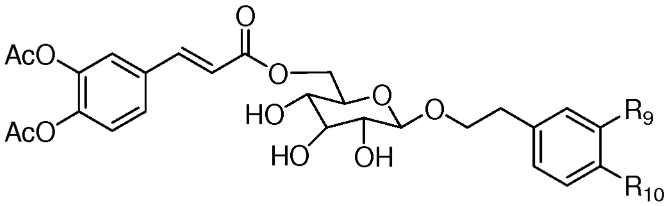

(V-2),

in formula (V-2), $R_9$ and $R_{10}$ being independently selected from hydrogen, a methoxy group, or a benzyloxy group, formula (V-3) is:

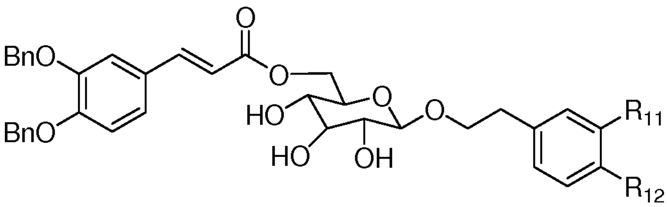

(V-3),

in formula (V-3), $R_{11}$ and $R_{12}$ being independently selected from hydrogen, a methoxy group, or a benzyloxy group, and formula (V-4) is:

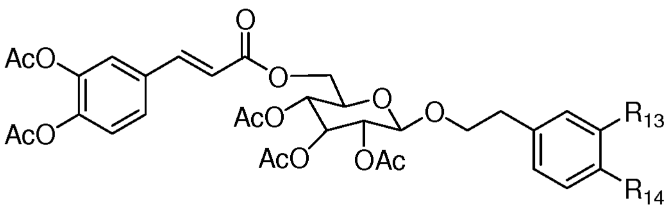

(V-4),

in formula (V-4), $R_{13}$ and $R_{14}$ being independently selected from hydrogen or chloride.

According to one embodiment of the present invention, the forming method further includes reacting the compound having the structure of formula (V-1) with copper(I) chloride and palladium dichloride in a mixture of methanol and water to form a compound having a structure of formula (VI-1), which formula (VI-1) is:

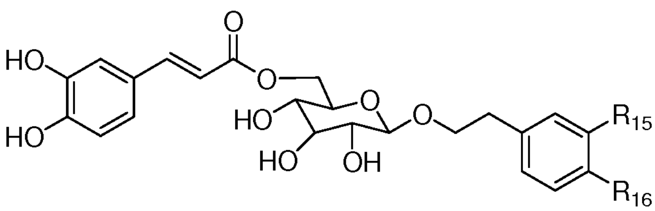

(VI-1),

in formula (VI-1), $R_{15}$ and $R_{16}$ being independently selected from hydrogen or a hydroxyl group.

According to one embodiment of the present invention, the forming method further includes reacting the compound having the structure of formula (V-2) with methylamine in methanol to form a compound having a structure of formula (VI-1), wherein formula (VI-1) is:

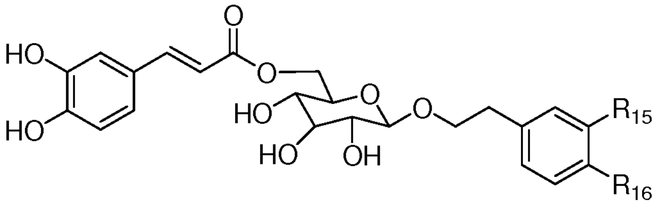
(VI-1),

in formula (VI-1), $R_{15}$ and $R_{16}$ being independently selected from hydrogen, chloride, a methoxy group, or a benzyloxy group.

According to one embodiment of the present invention, the forming method further includes reacting the compound having the structure of formula (V-4) with methylamine in methanol to form a compound having a structure of formula (VI-2), wherein formula (VI-2) is:

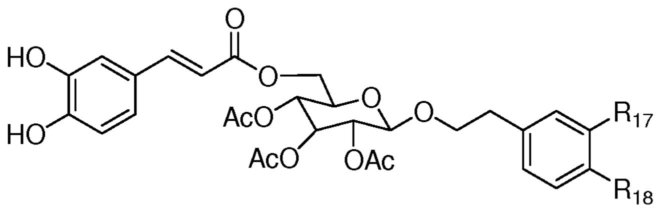
(VI-2),

in formula (VI-2), $R_{17}$ and $R_{18}$ being independently selected from hydrogen or chloride.

The isoacteoside derivative of the present invention modifying the chemical structure of isoacteoside equips the drug including the isoacteoside derivative of the present invention with uses of treating or preventing the amyloid-related disease and preventing the eye disease.

## BRIEF DESCRIPTION OF THE DRAWINGS

The invention can be more fully understood by reading the following detailed description of the embodiment, with reference made to the accompanying drawings as follows:

Figs. 1A-1D are result diagrams of the isoacteoside derivative of the embodiments of the present invention in inhibiting amyloid accumulation and cell viability;

Figs. 2A-2B are result diagrams of the isoacteoside derivative of the embodiments of the present invention in inhibiting amyloid accumulation and cell viability, respectively;

Figs. 3A-3B are result diagrams of the isoacteoside derivative of the embodiments of the present invention in inhibiting amyloid accumulation;

Figs. 4A-4B are result diagrams of the isoacteoside derivative of the embodiments of the present invention in inhibiting amyloid accumulation;

Fig. 5 is a degradation result diagram of the isoacteoside derivative of the embodiments of the present invention; and

Figs. 6A-6B are result diagrams of the isoacteoside derivative of the embodiments of the present invention in preventing eye disease.

## DETAILED DESCRIPTION

The detailed description provided below is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

An aspect of the present invention provides an isoacteoside derivative, having a structure of formula (I):

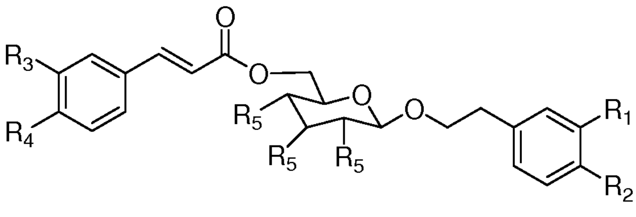

(I),

in formula (I), $R_1$ and $R_2$ being independently selected from hydrogen, halogen, a hydroxy group, or a hydrocarboxyl group, $R_3$ and $R_4$ being independently selected from a hydroxy group, a hydrocarboxyl group, or an acyloxy group, and $R_5$ being independently selected from a hydroxy group or an acyloxy group.

It is noteworthy that the "hydrocarboxyl group" described herein represents a group generated by bonding a carboxyl group and oxygen ions, which the carboxyl group is an organic compound composed by carbon and hydrogen, including alkanes, alkenes, alkynes, cyclic hydrocarbons, and aromatic hydrocarbons. "Acyloxy group" represents a group generated by bonding an acyl group and oxygen ions, which the acyl group represents a functional group derived by the removal of one or more hydroxyl groups from an oxoacid.

Further, the "amyloid-related disease" described herein represents neurodegeneration, Alzheimer's disease, mild Cognitive Impairment, Lewy body dementia, Down's syndrome, hereditary cerebral hemorrhage with amyloidosis (Dutch type), the Guam Parkinson-Dementia complex, progressive supranuclear palsy, multiple sclerosis, Creutzfeld Jacob disease, Parkinson's disease, frontotemporal dementia, Pick's disease, amyotrophic lateral sclerosis, inclusion-body myositis, adult-onset diabetes, senile cardiac amyloidosis, or endocrine tumor.

Moreover, the "eye disease" described herein represents neuronal degeneration, visual cortical defect, glaucoma, cataract, ocular amyloidosis, macular degeneration, optic nerve drusen, optic neuropathy, optic neuritis, or lattice corneal dystrophy.

In an embodiment of the present invention, when at least one of $R_1$ and $R_2$ is the halogen, the at least one of $R_1$ and $R_2$ is chloride.

In an embodiment of the present invention, when at least one of $R_1$ and $R_2$ is the hydrocarboxyl group, the at least one of $R_1$ and $R_2$ is independently selected from an alkoxy group, an alkenyloxy group, or an aryloxy group.

It is noteworthy that the "alkoxy group" described herein represents a group generated by bonding an alkyl group and oxygen ions. The "alkenyloxy group" described herein represents a group generated by bonding an alkenyl group and oxygen ions. The "aryloxy group" described herein represents a group generated by bonding an aryl group and oxygen ions, which the aryl group represents a functional group derived from any aromatic rings.

In an embodiment of the present invention, when at least one of $R_1$ and $R_2$ is the alkoxy group, the at least one of $R_1$ and $R_2$ is a methoxy group. The methoxy group has a structure of $-O-CH_3$, and is indicated as "OMe" in the following formulas.

In an embodiment of the present invention, when at least one of $R_1$ and $R_2$ is the alkenyloxy group, the at least one of $R_1$ and $R_2$ is an allyloxy group.

The allyloxy group has a structure of

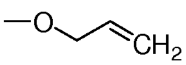

,

and is indicated as "OAll" in the following formulas.

In an embodiment of the present invention, when at least one of $R_1$ and $R_2$ is the aryloxy group, the at least one of $R_1$ and $R_2$ is a benzyloxy group.

The benzyloxy group has a structure of

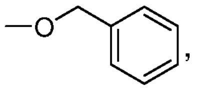

and is indicated as "OBn" in the following formulas.

In an embodiment of the present invention, when at least one of $R_3$ and $R_4$ is the hydrocarboxyl group, the at least one of $R_3$ and $R_4$ is independently selected from an alkenyloxy group or an aryloxy group.

In an embodiment of the present invention, when at least one of $R_3$ and $R_4$ is the alkenyloxy group, the at least one of $R_3$ and $R_4$ is an allyloxy group.

In an embodiment of the present invention, when at least one of $R_3$ and $R_4$ is the aryloxy group, the at least one of $R_3$ and $R_4$ is a benzyloxy group.

In an embodiment of the present invention, when at least one of $R_3$ and $R_4$ is the acyloxy group, the at least one of $R_3$ and $R_4$ is an acetoxy group. The acetoxy group has a structure of

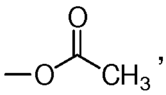

and is indicated as "OAc" in the following formulas.

In an embodiment of the present invention, $R_3$ and $R_4$ are the same substituent.

In an embodiment of the present invention, when $R_5$ is the acyloxy group, $R_5$ is an acetoxy group.

In an embodiment of the present invention, $R_5$ are the same substituent.

In an embodiment of the present invention, the isoacteoside derivative is selected from following structures:

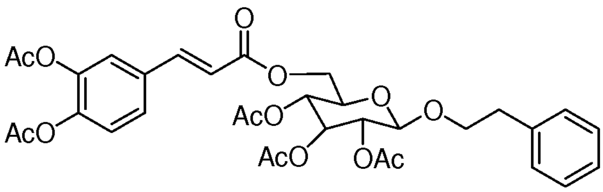
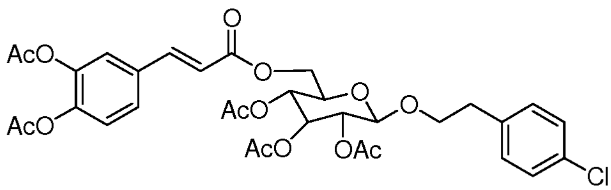

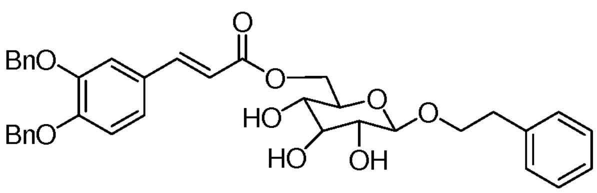
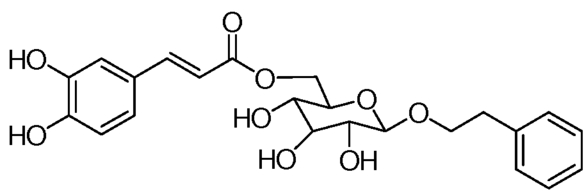

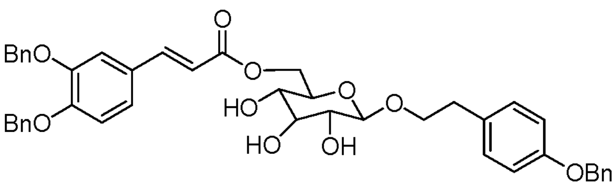
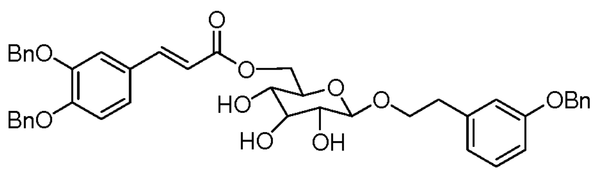

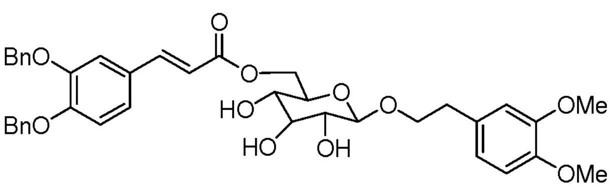
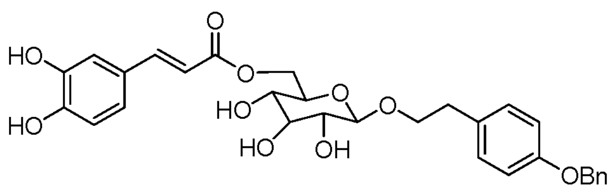

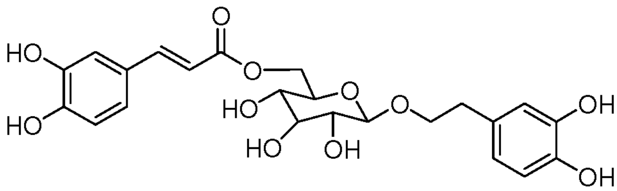

Another aspect of the present invention provides a use of a medicine for preventing or treating an amyloid-related disease, which the medicine is prepared from the aforementioned isoacteoside derivative.

According to one embodiment of the present invention, the amyloid is β-amyloid peptide (Aβ.

β-amyloid peptide is an amyloid precursor protein (APP), and by reactions of different secretases, the β-amyloid peptide with about 37-49 amino acids is formed from a protein originally with 770 amino acids. $A\beta_{40}$ and $A\beta_{42}$ are commonly seen β-amyloid peptide, which β-amyloid plaque is more easily formed for $A\beta_{42}$ comparing to $A\beta_{40}$due to its higher hydrophobicity. The accumulated Aβ has cytotoxicity, which may induce a series of complex reaction, such as synaptic change, Tau protein phosphorylation, neurotransmitter reduction, glial cell proliferation, and inflammatory reactions. These reactions may cause a series of pathological damage, such as plaque formation and neurofibrillary tangle, resulting in neuronal degeneration and dysfunction, or even death, and eventually leading to neurodegenerative diseases. β-amyloid plaque accumulation is considered to be one of the causes of Alzheimer's disease, and therefore most development of related medicine for treating or preventing the occurrence of Alzheimer's disease and worsening of symptoms currently is mainly to interfere the production pathway of the β-amyloid peptide. By reducing the generation of β-amyloid peptide, inhibiting the accumulation of β-amyloid peptide extracellularly, and inhibiting the aggregation of β-amyloid peptide, the formation of β-amyloid plaques can be prevented. The isoacteoside derivative of the present invention can inhibit the aggregation, and thereby having effects of neuroprotection, treating neurodegenerative diseases, etc.

Yet another aspect of the present invention provides a use of a medicine for preventing an eye disease, which the medicine is prepared from the aforementioned isoacteoside derivative.

As individual ages, the accumulation of oxidative damage caused by free radicals to cell DNA, proteins, lipids, and other cellular macromolecules can cause aging, which the degeneration of the retina and the central nervous system are believed to be highly correlated to oxidative stress damage.

Retinal pigment epidermis (RPE) cell is located between neuroepithelial layer of retina and choroid, and has a variety of physiological functions, such as retinal barrier, phagocytosis, involving in metabolism of visual cycle, antioxidant, and secretion of growth factors. Like other tissues, retinal pigment epidermis cell is susceptible to oxidative stress damage, which causes cell death and leads to retinopathy, visual dysfunction, or loss of visual function in severe cases. Therefore, retinal pigment epidermis cell is often used as cell model for retinopathy diseases, such as diabetic retinopathy and age-related macular degeneration. The isoacteoside derivative of the present invention can reduce the free radicals, and prevent oxidative stress damage, and thereby having effects of antioxidant, protecting the retinal cells, preventing the eye disease, etc.

The isoacteoside derivative of the present invention modifies the chemical structure of isoacteoside to have the effects of treating or preventing the amyloid-related disease, neuroprotection, treating neurodegenerative disease, preventing the eye disease, etc.

The following provides several examples to describe the method of the present invention in greater detail, however, it is intended as a exemplary description, and is not intended to limit the present invention. The protection scope of the present invention depends on the appended claim.

**Synthesis of isoacteoside derivative**

Procedures A-G were used to synthesis the isoacteoside derivative of the embodiments.

Procedure A included the following steps:

1. At room temperature (r.t), 5 mmol of boron trifluoride-diethyl etherate ($BF_3Et_2O$) solution was added to 25 mL of dichloromethane (DCM) solution, which included 5 mmol of β-D-glucose pentaacetate, and respectively included 10 mmol of compounds 1a-1f having structures of formula (II). Formula (II) was:

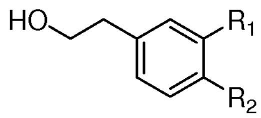

(II),

which the compound 1a's $R_1$ was hydrogen, and $R_2$ was hydrogen; the compound 1b's $R_1$ was hydrogen, and $R_2$ was chloride; the compound 1c's $R_1$ was a methoxy group (OMe), and $R_2$ was a methoxy group; the compound 1d's $R_1$ was hydrogen, and $R_2$ was a benzyloxy group (OBn); the compound 1e's $R_1$ was a benzyloxy group, and $R_2$ was a benzyloxy group; and the compound 1f's $R_1$ was a benzyloxy group, and $R_2$ was hydrogen.

2. The mixture of step 1 was stirred for 6 hours, and then was vigorously stirred with saturated aqueous sodium bicarbonate solution for 30 min.

3. The combined extracts of step 2 were dried, filtered and evaporated. Then, the residue was purified by silica gel using a solvent mixture of ethyl acetate/n-hexane (EA: n-hexane = 1:4, v/v) to give compounds 2a-2f having structures of formula (III), which the yield for compound 2a was 45%. Formula (III) was:

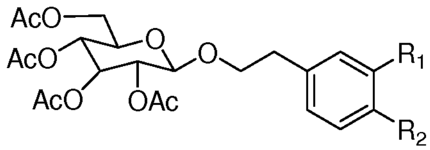

(III),

which the compound 2a's $R_1$ was hydrogen, and $R_2$ was hydrogen; the compound 2b's $R_1$ was hydrogen, and $R_2$ was chloride; the compound 2c's $R_1$ was a methoxy group, and $R_2$ was a methoxy group; the compound 2d's $R_1$ was hydrogen, and $R_2$ was a benzyloxy group; the compound 2e's $R_1$ was a benzyloxy group, and $R_2$ was a benzyloxy group; and the compound 2f's $R_1$ was a benzyloxy group, and $R_2$ was hydrogen.

The reaction process of Procedure A was as follows:

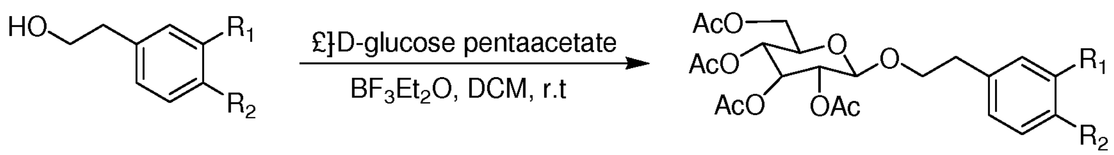


Procedure B included the following steps:

1. 2 mmol of the compounds 2d-2f were respectively mixed with 10% of palladium on carbon (Pd/C) in methanol (MeOH), which was stirred at room temperature under hydrogen ($H_2$) for 6 hours. Then, the catalyst was filtered off, and the filtrate was concentrated *in vacuo* to give a yellow residue. The residue was purified by silica gel using a solvent mixture of ethyl acetate/n-hexane (EA: n-hexane = 1:1, v/v) to give an intermediate product.

2. The intermediate product of step 1 was mixed with potassium carbonate, allyl bromide, and acetone. The mixture was refluxed under a $CaCl_2$ drying tube in a silicone oil bath for 10 hours and then cooled to room temperature.

3. The insoluble salts of step 2 were filtered, and washed with DCM.

Then, the filtrate and DCM were evaporated.

4. The crude product of step 3 was stirred in 10% of potassium hydroxide-methanol (KOH-MeOH) solution for 30 minutes. Then, the mixture was evaporated under reduced pressure. The residue was purified by silica gel using a solvent mixture of ethyl acetate/n-hexane (EA: n-hexane = 1:2, v/v) to give compounds 3d-3f having structures of formula (IV-1). Formula (IV-1) was:

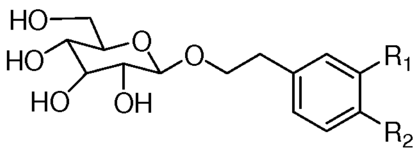

(IV-1),

which the compound 3d's $R_1$ was hydrogen, and $R_2$ was an allyloxy group (OAll); the compound 3e's $R_1$ was an allyloxy group, and $R_2$ was an allyloxy group; and the compound 3f's $R_1$ was an allyloxy group, and $R_2$ was hydrogen.

The reaction process of Procedure B was as follows:

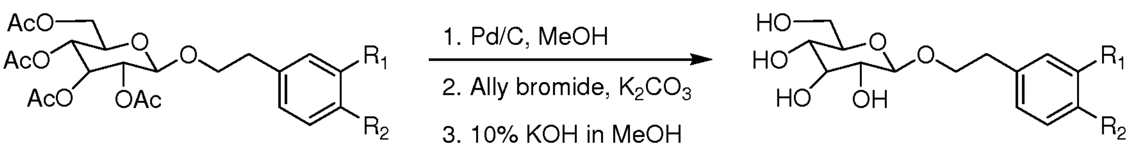


Procedure C included the following steps:

1. 1.5 mmol of sodium methoxide (NaOMe) was respectively added to 3 mmol of compounds 2a-2f in 15 mL of methanol solution. The mixture was stirred at room temperature for 30 minutes.
2. The mixture of step 1 was evaporated under reduced pressure. The residue was purified by silica gel using a solvent mixture of ethyl acetate/n-hexane (EA: n-hexane = 1:4, v/v) to give compounds 3a-3c and 3g-3i having the structures of formula (IV-1), which the yield for compound 3a was 85%. The compound 3a's $R_1$ was hydrogen, and $R_2$ was hydrogen; the compound 3b's $R_1$ was hydrogen, and $R_2$ was chloride; the compound 3c's $R_1$ was a methoxy group, and $R_2$ was a methoxy group; the compound 3g's $R_1$ was hydrogen, and $R_2$ was a benzyloxy group; the compound 3h's $R_1$ was a benzyloxy group, and $R_2$ was a benzyloxy group; and the compound 3i's $R_1$ was a benzyloxy group, and $R_2$ was hydrogen.

The reaction process of Procedure C was as follows:

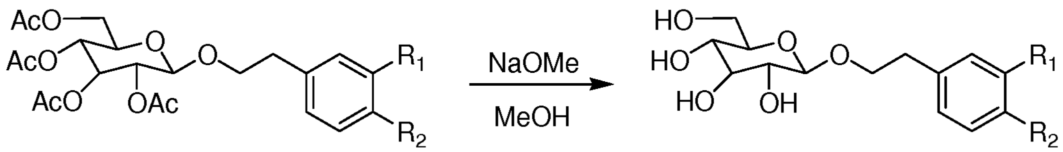


Procedure D included the following steps:

1. 0.01 mmol of acetyl chloride was respectively added to a solution with 2 mmol of compound 2a or 2b in 10 mL of 1:1 methanol-dichloromethane (MeOH/DCM). The mixture was stirred at room temperature for 48 hours, and at the end of which time, thin layer chromatography (TLC) was used to indicate the reaction was complete.
2. The mixture of step 1 was neutralized with triethanolamine (TEA).

Then, the reaction mixture was concentrated, and the residue was passed through a silica gel column with ethyl acetate (EA) as the eluent to give compounds 3j and 3k having the structures of formula (IV-2). Formula (IV-2) was:

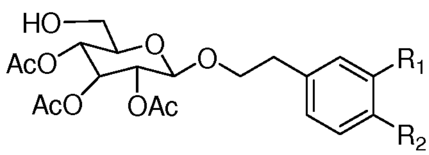
(IV-2),

which the compound 3j's $R_1$ was hydrogen, and $R_2$ was hydrogen; and the compound 3k's $R_1$ was hydrogen, and $R_2$ was chloride.

The reaction process of Procedure D was as follows:

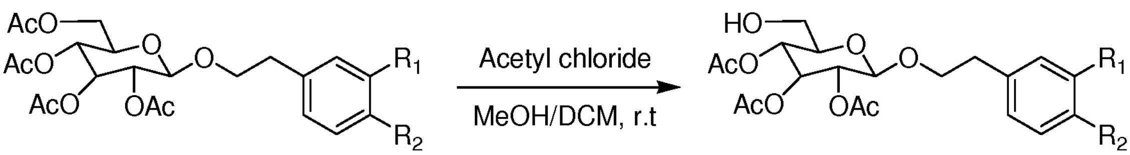

Procedure E included the following steps:

1. Based on different compounds, step 1 of Procedure included the following three conditions:

a. 2 mmol of compounds 3a-3c, 3g, 3h, 3j, and 3k were respectively added at 0ºC to a solution with 2.2 mmol of di-O-acetylferulic acid chloride, dichloromethane, and 1.5 mL of pyridine.
b. 2 mmol of compounds 3d-3f were respectively added at 0ºC to a solution with 2.2 mmol of di-O-allylferulic acid chloride, dichloromethane, and 1.5 mL of pyridine.
c. 2 mmol of compounds 3a, 3c, 3g, and 3i were respectively added at 0ºC to a solution with 2.2 mmol of di-O-benzylferulic acid chloride, dichloromethane, and 1.5 mL of pyridine. The mixture was stirred at 10ºC for 10 hours. The solvent was evaporated and the residue was dissolved in ethyl acetate.

2. The organic layer of the intermediate product of step 1 was washed successively with water and brine, and dried with $MgSO_4$ and evaporated. The residue was purified by silica gel using a solvent mixture of ethyl acetate/n-hexane (EA: n-hexane = 1:1, v/v) to give compounds 4d-4f having the structures of formula (V-1), compounds 4a-4c, 4g, and 4h having the structures of formula (V-2), compounds 4i and 4m-4o having the structures of formula (V-3), and compounds 4j and 4k having the structures of formula (V-4), which the yield for compound 4a was 43%.

Formula (V-1) was:

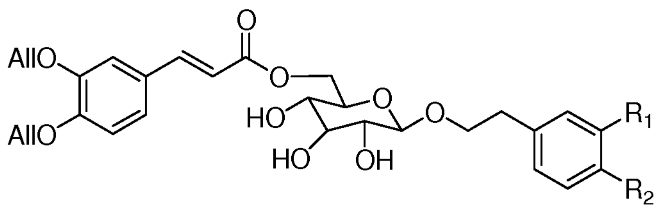
(V-1),

which the compound 4d's $R_1$ was hydrogen, and $R_2$ was an allyloxy group; the compound 4e's $R_1$ was an allyloxy group, and $R_2$ was an allyloxy group; and the compound 4f's $R_1$ was an allyloxy group, and $R_2$ was hydrogen.
Formula (V-2) was:

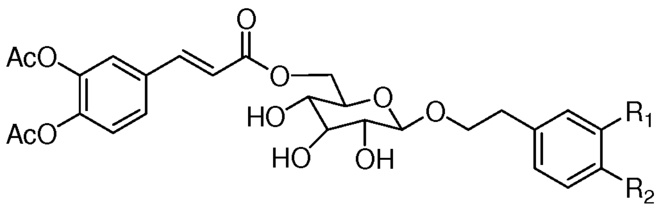
(V-2),

which the compound 4a's $R_1$ was hydrogen, and $R_2$ was an hydrogen; the compound 4b's $R_1$ was hydrogen, and $R_2$ was chloride; the compound 4c's $R_1$ was a methoxy group, and $R_2$ was a methoxy group; the compound 4g's $R_1$ was hydrogen, and $R_2$ was a benzyloxy group; and the compound 4h's $R_1$ was a benzyloxy group, and $R_2$ was a benzyloxy group.
Formula (V-3) was:

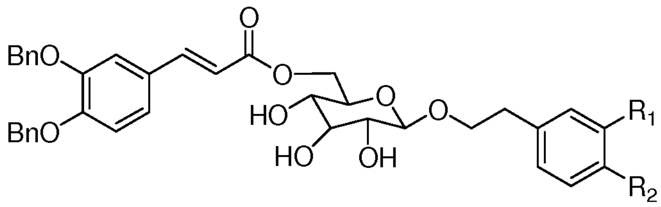
(V-3),

which the compound 4i's $R_1$ was a benzyloxy group, and $R_2$ was hydrogen; the compound 4m's $R_1$ was hydrogen, and $R_2$ was a benzyloxy group; the compound 4n's $R_1$ was hydrogen, and $R_2$ was hydrogen; and the compound 4o's $R_1$ was a methoxy group, and $R_2$ was a methoxy group.

Formula (V-4) was:

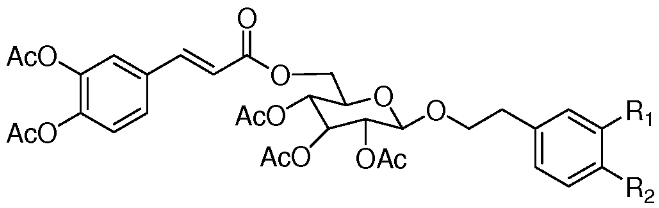

(V-4),

which the compound 4j's $R_1$ was hydrogen, and $R_2$ was hydrogen; and the compound 4k's $R_1$ was hydrogen, and $R_2$ was chloride.

The reaction process of Procedure E for forming the compounds 4d-4f was as follows:

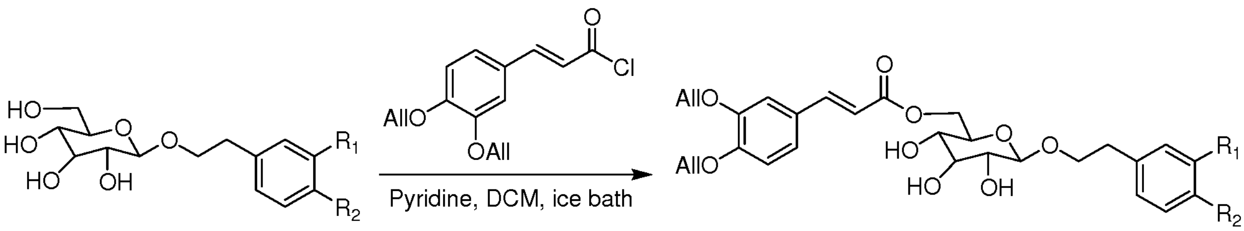


; the reaction process for forming the compounds 4a-4c, 4g, and 4h was as follows:

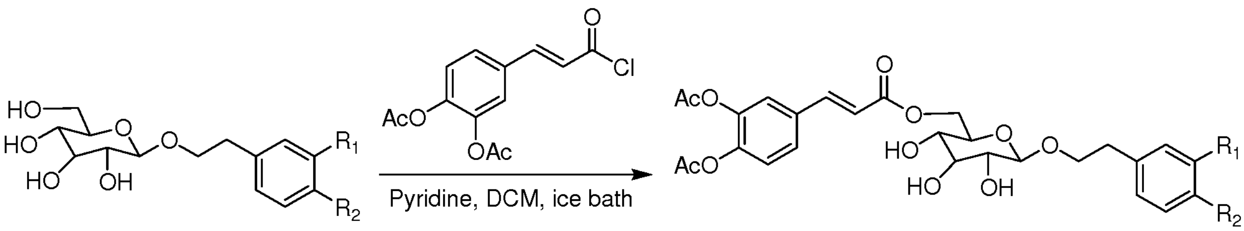


; the reaction process for forming the compounds 4i and 4m-4o was as follows:

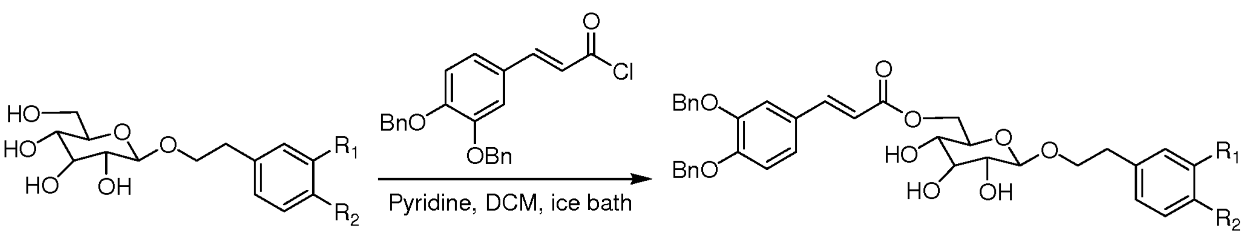


; and the reaction process for forming the compounds 4j and 4k was as follows:

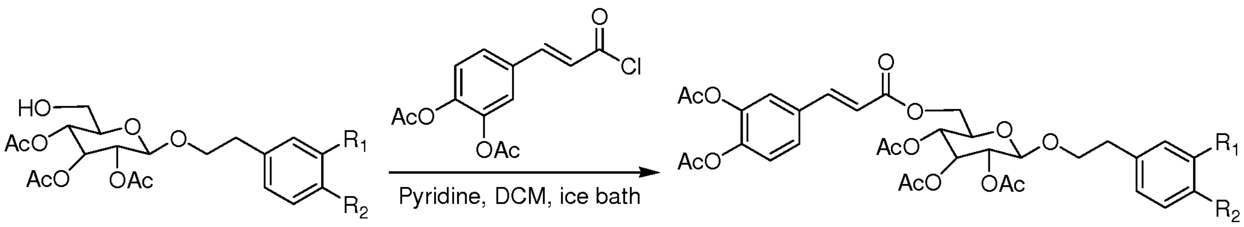


Procedure F included the following steps:

1. The compounds 4d-4e were respectively mixed with copper(I) chloride (CuCl) and palladium(II) chloride ($PdCl_2$) in methanol and water , and was stirred strongly at room temperature to give compounds 5d-5f having the structures of formula (VI-1). Formula (VI-1) was:

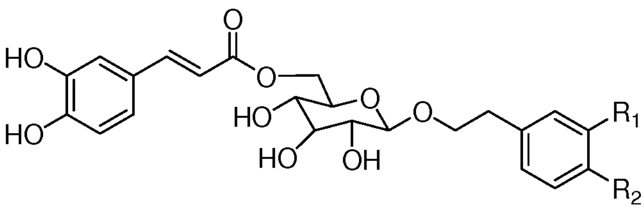
(VI-1),

which the compound 5d's $R_1$ was hydrogen, and $R_2$ was a hydroxyl group; the compound 5e's $R_1$ was a hydroxyl group, and $R_2$ was a hydroxyl group; and the compound 5f's $R_1$ was a hydroxyl group, and $R_2$ was hydrogen.

The reaction process of Procedure F for forming the compounds 5d-5f was as follows:

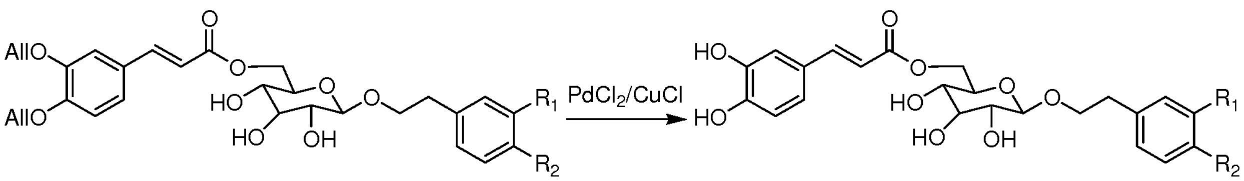


Procedure G included the following steps:

1. 1 mL of 40% methylamine in methanol was added to a solution respectively with 2 mmol of the compounds 4a-4c, 4g, 4h, 4j, and 4k in dichloromethane at 10ºC.
2. The reaction mixture of step 1 was stirred for 20 minutes and then concentrated *in vacuo.* The residue was purified by silica gel using a solvent mixture of methanol/dichloromethane (1:20, v/v) to give compounds 5a-5c, 5g, and 5h having the structures of formula (VI-1) and compounds 5j and 5k having the structures of formula (VI-2), which the yield for the compound 5a was 90%. The compound 5a's $R_1$ was hydrogen, and $R_2$ was an hydrogen; the compound 5b's $R_1$ was hydrogen, and $R_2$ was chloride; the compound 5c's $R_1$ was a methoxy group, and $R_2$ was a methoxy group; the compound 5g's $R_1$ was hydrogen, and $R_2$ was a benzyloxy group; and the compound 5h's $R_1$ was a benzyloxy group, and $R_2$ was a benzyloxy group. Formula (VI-2) was:

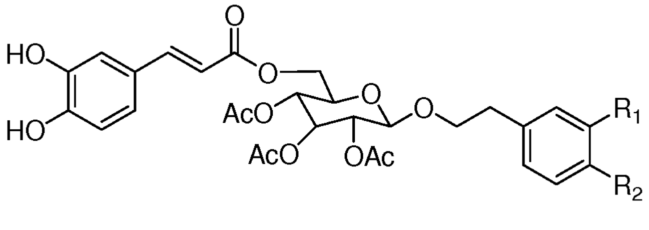
(VI-2),

which the compound 5j's $R_1$ was hydrogen, and $R_2$ was hydrogen; and the compound 5k's $R_1$ was hydrogen, and

$R_2$ was chloride.

The reaction process of Procedure G for forming the compounds 5a-5c, 5g, and 5h was as follows:

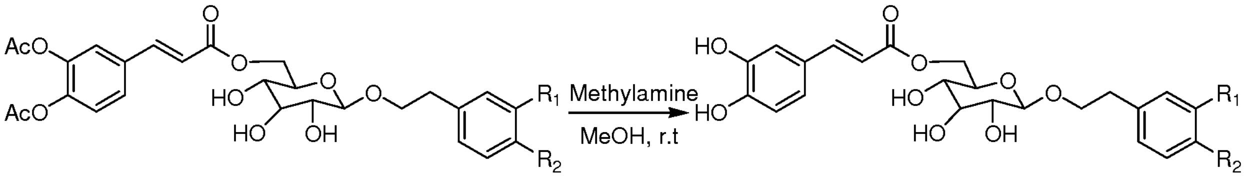


; and the reaction process for forming the compounds 5j and 5k was as follows:

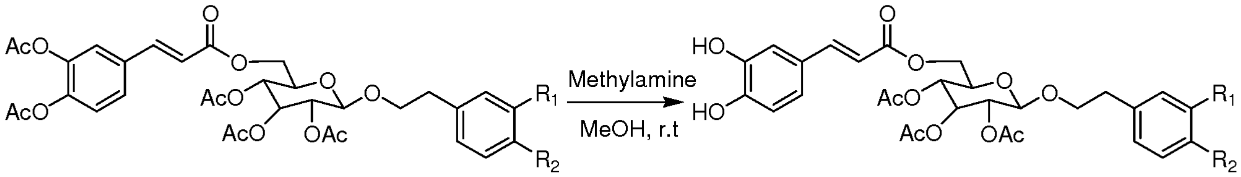


.

The following experimental examples used samples 1-9 listed in the following Table 1 to prepare solutions with different concentrations to conduct various experiments.

Table 1

| | |
|---|---|
| Sample 1 (Compound 4j) | Sample 5 (Compound 4m) |
| 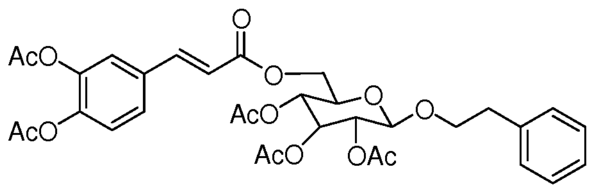 | 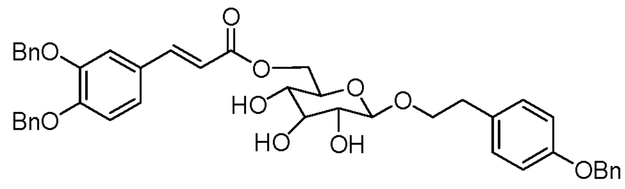 |
| Sample 2 (Compound 4k) | Sample 6 (Compound 4i) |
| 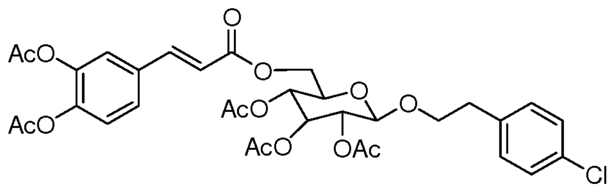 | 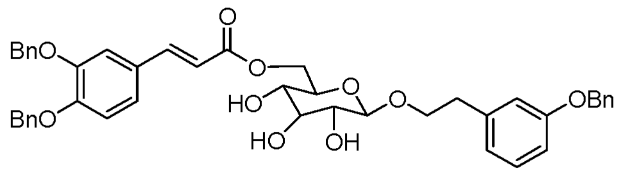 |
| Sample 3 (Compound 4n) | Sample 7 (Compound 4o) |
| 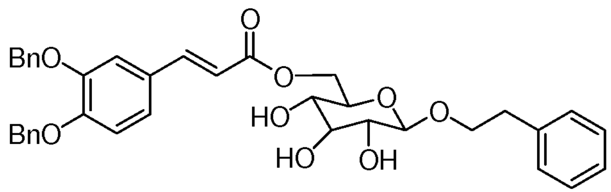 | 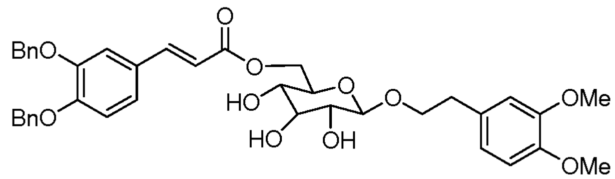 |
| Sample 4 (Compound 5a) | Sample 8 (Compound 5g) |
| 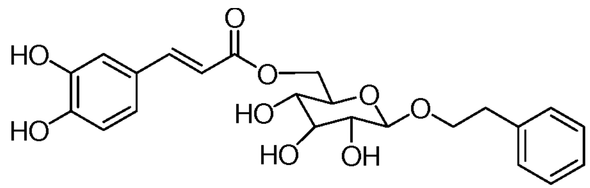 | 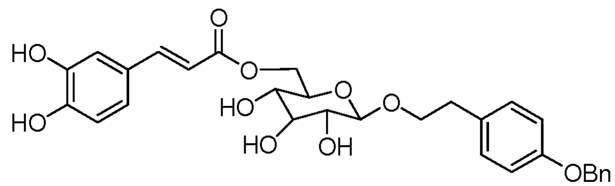 |
| Sample 9 (Compound 5e) | |

(continued)

| | |
|---|---|
| 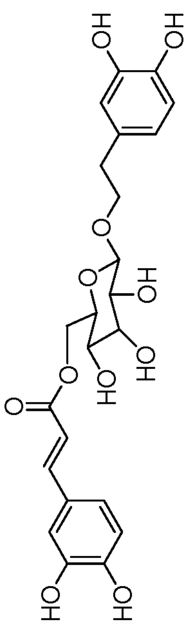 | |

4o $^{1}$HNMR ($CDCl_3$) δ7.60 (d, 1 H, *J*=18), δ7.43-7.21 (m, 15H), δ7.31-7.25 (m, 3H) δ6.77 (d, 1 H, *J*=9), δ6.29 (d, 1 H, *J*=18), δ5.16 (d, 4H, J=9), δ4.64-4.40 (m, 1 H), δ4.36-4.11 (m, 2H), δ3.76-3.35 (m, 6H), δ2.94 (t, 2H, J=6).

4i$^{1}$HNMR ($CDCl_3$) δ7.67 (d, 1 H, *J*=16), δ7.45-7.29 (m, 3H), δ7.24 (d, 1 H, J=12), δ6.8(s, 1 H), δ7.76 (s, 2H), δ6.53 (d, 1 H, *J*=16), δ 4.45-3.90 (m, 3H), δ3.90-3.81 (m, 1 H), δ3.78-3.74 (m, 8H), δ3.73-3.70 (m, 1 H), δ 3.60-2.86 (m, 5H).

4m$^{1}$HNMR ($CDCl_3$) δ7.60 (d, 1 H, *J*=18), δ7.43-6.84 (m, 22H), δ6.29 (d, 1 H, *J*=18), δ5.13 (s, 4H), δ4.97 (s, 2H), δ4.68-4.63 (m, 1 H), δ4.37-4.06 (m, 2H), δ3.73-3.36 (m, 6H), δ2.94 (t, 2H, J=6).

4n$^{1}$HNMR ($CDCl_3$) δ7.60 (d, 1 H, *J*=18), δ7.43-7.01 (m, 15H), δ7.41-7.45 (m, 3H) δ6.87 (d, 1 H, *J*=9), δ6.29 (d, 1 H, *J*=18), δ5.16 (d, 4H, J=9), δ4.64-4.40 (m, 1 H), δ4.36-4.11 (m, 2H), δ3.76-3.35 (m, 6H), δ2.94 (t, 2H, J=6).

5a$^{1}$HNMR ($CDCl_3$) δ7.34-7.21 (m, 7H), δ6.80-6.53 (m, 3H), δ5.02 (s, 2H), δ4.38-4.05 (m, 3H), δ3.73-4.3.70 (m, 1 H), δ3.22-2.92 (m, 3H), δ2.91-2.62 (m, 7H), δ2.62 (t, 2H, J=6).

First, samples 1-9 listed in Table 1 were accurately weighed respectively, and dimethyl sulfoxide (DMSO) was used as a solvent to prepare stocks with a concentration of 10mM. The molecular weight and weight of the samples and the volume of the solvent included in the stocks are listed in the following Table 2.

Table 2

| | Sample molecular weight | Sample weight (mg) | Solvent volume (mL) |
|---|---|---|---|
| Sample 1 | 656.6 | 10.1 | 1.53 |
| Sample 2 | 691.6 | 11.2 | 1.62 |
| Sample 3 | 626.4 | 10.8 | 1.72 |
| Sample 4 | 446.5 | 11.2 | 2.50 |
| Sample 5 | 732.4 | 11.8 | 1.61 |
| Sample 6 | 732.4 | 10.5 | 1.43 |
| Sample 7 | 686.7 | 11 | 1.60 |
| Sample 8 | 552.5 | 10.2 | 1.85 |
| Sample 9 | 478.45 | 20 | 4.18 |

Then, the stocks with the concentration of 10mM were used to prepare solutions with different sample concentrations. The stocks were used to prepare a concentration of 5 μM, which 0.5 μL of the stocks were diluted to 1 mL; to prepare a concentration of 10 μM, which 1 μL of the stocks were diluted to 1 mL; to prepare a concentration of 20 μM, which 2 μL of the stocks were diluted to 1 mL; to prepare a concentration of 50 μM, which 5 μL of the stocks were diluted to 1 mL; to prepare a concentration of 100 μM, which 1 μL of the stocks were diluted to 0.1 mL; and to prepare a concentration of 200 μM, which 2 μL of the stocks were diluted to 0.1 mL.

Experimental example 1 to Experimental example 4 used the following three aspects to evaluate the efficacy of the isoacteoside derivative of the present invention, including: 1) whether the formation of β-amyloid peptide (Aβ) in cell can be decreased; 2) whether by promoting the activity of the enzyme that is responsible for eliminating β-amyloid peptide, the efficiency of eliminating β-amyloid peptide can be thereby increased; and 3) whether the aggregation of $A\beta_{40}$ and $A\beta_{42}$ can be inhibited.

**Experimental example 1: Experiment on inhibiting $A\beta_{40}$ accumulation (I)**

This experiment was divided into two stages: The first stage used a lower sample concentration for preliminary screening; the second stage was based on the result of the first stage to choose effective samples and increased the testing concentration of the samples in order to obtain the best concentration and the best result of sample for inhibiting $A\beta_{40}$ accumulation without affecting the cytotoxicity.

Experimental method: Human neuroblastoma cell (SH-SY5Y-APP695), which expresses Swedish APP695 mutation, was incubated in a 3.5-cm culture dish until the cell concentration reached 90% full. When passage, each well of a 24-well plate was seeded with 4 x $10^5$ cells. The culture medium was replaced with 300 μL of chemical-defined medium, which is a DMEM/F12 medium including 5 mM of Hepes buffer, 0.6% of glucose, 3 mM of $NaHCO_3$, 2.5 μM of glutamine, 100 pg/mL of transferrin, 20 nM of progesterone, 60 μM putrescine, 30 nM of sodium selenite, and 2 pg/mL of heparin, the next day. 3 μL of the testing sample was added into each well, and each concentration of each sample included 4 groups. After the cells were placed in an incubator (37°C, 5% $CO_2$) and treated with the samples for 24 hours, the culture medium was collected and analyzed the amount of $A\beta_{40}$ in the culture medium after treated by the samples through Human $A\beta_{1-40}$ Immunoassay kits (Cat.KHB3482, Life Technologies). The cells treated by the samples were

analyzed by MTT assay to evaluate the toxicity to the cells caused by sample treatment.

The testing concentrations of Sample 1 were 5 μM and 10 μM, and the testing concentrations of Samples 2-8 were 10 μM and 20 μM. The amount of $A\beta_{40}$ in a SH-SY5Y-APP695 cell medium that there was no sample added as a control and set to 100%, and the amounts of $A\beta_{40}$ in the medium after respectively treated by the testing samples were compared to the control and expressed in percentage. β-secretase inhibitor (β-SI) was used as a positive control. The experiment results are shown in Figs. 1A-1D, which Figs. 1A and 1C are result diagrams of the samples in inhibiting $A\beta_{40}$ accumulation, and Figs.1B and 1D are result diagrams of the cell viability after treated by the samples. The results shown in Figs 1A-1D are mean ± standard error of four experimental groups (n = 4), and the statistical differences between the control and the testing samples were analyzed by Dunnett's multiple comparison test, which "*" represented $p < 0.05$; "**" represented $p < 0.01$; "***" represented $p < 0.001$; and "****" represented $p < 0.0001$.

Experimental example 1 used a lower sample concentration for preliminary screening, and the results showed in Figs 1A-1D suggested that Sample 3, 4, and 6 had effects of inhibiting $A\beta_{40}$ accumulation, which Sample 3 had the best result of inhibiting $A\beta_{40}$ accumulation, and the efficacy of inhibiting $A\beta_{40}$ accumulation of Sample 3 increased as the sample concentration increased.

**Experimental example 2: Experiment on inhibiting $A\beta_{40}$ accumulation (II)**

Based on the experiment results of Experimental example 1, Sample 3, 4, and 6 having efficacy of inhibiting $A\beta_{40}$ accumulation were selected. The testing concentrations of the samples were increased to 20, 50, and 100 μM, and were experimented using the method of Experimental example 1. Each concentration of each sample included 4 groups, and the experiment results are shown in Figs. 2A-2B. Fig. 2A is a result diagram of the samples in inhibiting $A\beta_{40}$ accumulation, and Fig. 2B is a result diagram of the cell viability after treated by the samples. The results shown in Figs 2A-2B are mean ± standard error of four experimental groups (n = 4), and the statistical differences between the control and the testing samples were analyzed by Dunnett's multiple comparison test, which "*" represented $p < 0.05$; "**" represented $p < 0.01$; "***" represented $p < 0.001$; and "****" represented $p < 0.0001$.

In Experimental example 2, the concentrations of Samples 3, 4, and 6 were increased in order to obtain the best concentration and the best result of the samples for inhibiting $A\beta_{40}$ accumulation in a condition of not affecting the cytotoxicity. The results showed in Figs. 2A-2B suggested that in the condition of not affecting the cytotoxicity, Sample 3 could inhibit about 20% of accumulation in the concentration of 20 μM, and Sample 6 could inhibit about 40% of accumulation in the concentration of 50 μM, while increasing the sample concentration could not further enhance the efficacy of inhibiting Aβ accumulation for Sample 4.

Therefore, based on the results of Experimental example 1 and Experimental example 2, the isoacteoside derivative of the embodiments of the present invention does have the efficacy of inhibiting $A\beta_{40}$ accumulation.

**Experimental example 3: Experiment on inhibiting Aβ aggregation (I)**

Experimental example 3 was to confirm the efficacy of the samples on inhibiting $A\beta_{40}$ and $A\beta_{42}$ aggregation.

$A\beta_{40}$ aggregation: The $A\beta_{40}$ stock was redissloved in DMSO to 10 mg/mL. Each group included 0.5 μL of 10 mg/mL $A\beta_{40}$ and 4.5 μL of the testing sample diluted with Dulbecco's Phosphate-Buffered Saline (D-PBS). The concentrations of Sample 1 were 10 μM and 100 μM, and the concentrations of Sample 2-8 were 20 μM and 200 μM. The total reaction volume was 5 μL, and each concentration of each sample included 6 groups. After incubating in a 37ºC incubator for 4 hours, 200 μL of thioflavin T working solution (ThT working solution) was added and mixed thoroughly, which the thioflavin T working solution was 10 μM of thioflavin T dissolving in potassium phosphate buffer (PB buffer, pH 6.0). 200 μL of the mixture was placed into a black, clear bottom 96-well plate, and the ThT fluorescence intensity (Ex: 440 nm, Em: 482 nm) was measured to determine the level of $A\beta_{40}$ aggregation. This experimental example used thioflavin T assay (ThT assay) to evaluate the level of $A\beta_{40}$ aggregation. Since ThT and Congo red derivatives can form bonds with aggregated form of Aβ protein, the level of Aβ aggregation is higher when the amount of bonded ThT is more. By detecting the variation in the amount of ThT, the change in the level of Aβ aggregation can be estimated. The value of not reacting with any sample (i.e. only containing 0.5 μL of $A\beta_{40}$ and 4.5 μL of D-PBS, which the final concentration of $A\beta_{40}$ was 1 mg/mL) was a control and set to 100%, and isoacteoside (IsoA) was used as a positive control. The values of the testing samples were compared to the control and expressed in percentage, and the experiment results are shown in Figs. 3A-3B. The results shown in Figs 3A-3B were mean ± standard error of six experimental groups (n = 6), and the statistical differences between the control and the testing samples were analyzed by Dunnett's multiple comparison test, which "*" represented $p < 0.05$; "**" represented $p < 0.01$; "***" represented $p < 0.001$; and "****" represented $p < 0.0001$.

Fig. 3A shows the experiment results of Samples 1-9 with a lower concentration in inhibiting $A\beta_{40}$accumulation. The concentration of Sample 1 was 10 μM, the concentration of Samples 2-9 was 20 μM, and the concentration of IsoA was 10 μg/mL. The measured value of the control without adding any sample was set to 100%, and other values were adjusted accordingly. Fig. 3B shows the experiment results of Samples 1-9 with a higher concentration in inhibiting

$A\beta_{40}$accumulation. The concentration of Sample 1 was 100 μM, the concentration of Samples 2-9 was 200 μM, and the concentration of IsoA was 100 μg/mL. The measured value of the control without adding any sample was set to 100%, and other values were adjusted accordingly. The experiment results shown in Fig. 3A suggested that in the lower concentration, Sample 2 could inhibit about 20% of $A\beta_{40}$aggregation in the concentration of 20 μM, and in the same concentration, Samples 4 and 8 could inhibit about 30% of $A\beta_{40}$ aggregation. The experiment results shown in Fig. 3B, which increased the concentrations of the samples and repeated the experiment, suggested that in the concentration of 200 μM, Samples 2 and 4 could inhibit about 20% of $A\beta_{40}$ aggregation, and Sample 8 could inhibit up to 70% of $A\beta_{40}$aggregation.

**Experimental example 4: Experiment on inhibiting Aβ aggregation (II)**

Experimental example 4 increased the testing concentrations of the samples to confirm the efficacy of the samples on inhibiting $A\beta_{42}$ aggregation.

Experimental method: $A\beta_{42}$ was redissloved in DMSO to 2.5 mg/mL, and Samples 1-9 were diluted with D-PBS to appropriate concentrations. The concentrations of Sample 1 were 10 μM and 100 μM, the concentrations of Sample 2-9 were 20 μM and 200 μM, and the concentrations of IsoA were 10 pg/mL and 100 μg/mL. Each reaction included 1 μL of $A\beta_{42}$ (final concentration was 0.25 mg/mL) and 9 μL of the testing sample, which each concentration of each sample included 8 groups, and was placed at 37°C reacting for 30 minutes after thoroughly mixed. 200 μL of Thioflavin T working solution was added and mixed thoroughly after the reaction completed, 200μL of the which was placed into a black, clear bottom 96-well plate, and the ThT fluorescence intensity (Ex: 440 nm, Em: 482 nm) was measured to determine the level of $A\beta_{42}$ aggregation. The value of not reacting with any sample (i.e. only containing 1 μL of $A\beta_{42}$ and 9 μL of D-PBS, which the final concentration of $A\beta_{42}$ was 0.25 mg/mL) was a control and set to 100%, and isoacteoside (IsoA) was used as a positive control. The values of the samples were compared to the control and expressed in percentage. The experiment results are shown in Figs. 4A-4B, which show mean ± standard error of eight experimental groups (n = 8), and the statistical differences between the control and the testing samples were analyzed by Dunnett's multiple comparison test, which "*" represented $p < 0.05$; "**" represented $p < 0.01$; "***" represented $p < 0.001$; and "****" represented $p < 0.0001$.

Fig. 4A shows the experiment results of Samples 1-9 with a lower concentration in inhibiting $A\beta_{42}$ accumulation. The concentration of Sample 1 was 10 μM, the concentration of Samples 2-9 was 20 μM, and the concentration of IsoA was 10 μg/mL. The level of $A\beta_{42}$ accumulation was measured by ThT assay, which the measured value of the control without adding any sample was set to 100%, and other values were adjusted accordingly. Fig. 4B shows the experiment results of Samples 1-9 with a higher concentration in inhibiting $A\beta_{42}$ accumulation. The concentration of Sample 1 was 100 μM, the concentration of Samples 2-9 was 200 μM, and the concentration of IsoA was 100 μg/mL. The level of $A\beta_{42}$ accumulation was measured by ThT assay, which the measured value of the control without adding any sample was set to 100%, and other values were adjusted accordingly.

The experiment results shown in Fig. 4A suggested that in the lower concentration, Sample 2 could inhibit about 20% of $A\beta_{42}$ aggregation in the concentration of 20 μM, and Samples 4 and 8 could inhibit about 50% and 60% of $A\beta_{42}$ aggregation in the concentration of 20 μM respectively. The experiment results shown in Fig. 4B suggested that after increasing the concentration of the samples, Sample 2 could inhibit about 40% of $A\beta_{42}$ aggregation in the concentration of 200 μM, Sample 4 could inhibit about 70% of $A\beta_{42}$ aggregation in the concentration of 200 μM, and Sample 8 could totally inhibit $A\beta_{42}$ aggregation in the concentration of 200 μM.

Therefore, based on the results of Experimental example 3 and Experimental example 4, the isoacteoside derivative of the embodiments of the present invention does have the efficacy of inhibiting $A\beta_{40}$accumulation and $A\beta_{42}$ accumulation.

Given the above, based on the results of Experimental example 1 to Experimental example 4, Samples 3, 4, and 6 could inhibit $A\beta_{40}$ formation in cell, which Sample 6 had the best result. As for evaluating whether the samples could inhibit Aβ aggregation, ThT assay was used to measure the level of Aβ aggregation. The experiment results showed that Samples 2, 4, and 8 could effectively inhibit $A\beta_{40}$and $A\beta_{42}$ accumulation, which Sample 8 had the best result. Sample 8 could inhibit about 70% of $A\beta_{42}$ aggregation and about 100% of $A\beta_{42}$ aggregation in the concentration of 200 μM.

In addition to β-amyloid peptide, the present invention also used the effects of different oxidative stress to retinal epithelial cells so as to observe the protective effect of the isoacteoside derivative of the present invention on retinal pigment epithelial cells.

**Experimental example 5: Experiment on Aβ degradation**

Experimental example 5 was to confirm that the samples could activate medicine for decomposing $A\beta_{40}$enzyme activity extracellularly to improve ability of enzymes for decomposing $A\beta_{40}$and to have effects of reducing extracellular

$A\beta_{40}$ level.

Experimental method: 2 x $10^7$ of mouse neuroblastoma cells (Neuro-2a) were placed on a T175 culture medium, and after overnight, the T175 culture medium was replaced with 30 mL of a chemical-defined medium incubating for 24 hours. After 24 hours, the chemical-defined medium incubated with the cells, which was called a conditioned medium, was centrifuged for 5 minutes at 13,000 rpm, and supernatant liquid was obtained. 10 ng of $A\beta_{40}$ and testing medicine were added to 300 μL of the conditioned medium, and the mixture was reacted at 37ºC for 24 hours. Immunoassay kits (Human $A\beta_{1-40}$ Immunoassay kits, Cat.KHB3482, Life Technologies) were used to measure the remaining amount of Aβ in each reaction to examine whether the medicine can improve the activity for enzyme in the medium to degrade Aβ. One not adding any testing medicine (i.e. only containing 10 ng of $A\beta_{40}$) was a control, and the measured level of Aβ was set to 100%. The levels of Aβ after treated by the testing medicine were compared to the control and expressed in percentage. The experiment results are shown in Fig. 5, which are mean ± standard error of four experimental groups (n = 4). The statistical differences between the control and the testing samples were analyzed by Dunnett's multiple comparison test, which "*" represented $p < 0.05$; "**" represented $p < 0.01$; "***" represented $p < 0.001$; and "****" represented $p < 0.0001$.

As the experiment results shown in Fig. 5, the level of $A\beta_{40}$ for not adding any medicine (C) was set to 100%, and other values were adjusted accordingly and presented in percentage. Sample 9 could effectively improve extracellular $A\beta_{40}$ degradation in the concentrations of 50 μM and 100 μM.

Therefore, based on the results of Experimental example 5, the isoacteoside derivative of the embodiments of the present invention does have the efficacy of degrading Aβ.

**Experimental example 6: Experiment on preventing eye disease**

Human retinal pigment epithelium cells (ARPE-19) were incubated in a DMEM/F12 cell culture medium (Life Technologies) containing 10 % of fetal bovine serum (FBS), and were passaged using a 96-well plate after the cell concentration reached 90% full, which each well was seeded with 4.5 x $10^3$ cells. Next day, Samples 2 and 8 were diluted with dimethyl sulfoxide (DMSO) to about 200 times of test concentration, and after that, appropriate amount of DMEM/F12 cell culture medium containing 5% fetal bovine serum was added and diluted to twice test concentration. Then, the resultant was mixed with 0.2 mM of tert-butyl hydroperoxide (tBHP, Sigma) in equal proportions to reach the test concentration (containing 0.5% of dimethyl sulfoxide). The diluted sample was added to the plate with the cells, which was placed in an incubator reacted for 24 hours, and the cell viability was analyzed by MTT solution. The absorbance was measured at a wavelength of 570 nm, and the cells not being treated by the medicine was a control, and the average absorbance of which was set to 100%. The cell viability of the cells being treated by the medicine was calculate by the following formula based on the measured absorbance:

$$\text{Cell viability} = (\text{absorbance of the experimental group/average absorbance of the control}) \times 100\%.$$

The tert-butyl hydroperoxide is an organic peroxide, and can be metabolized by free radicals, which causes lipid oxidation covalently bonded with cellular molecules resulting in cell damage. Therefore, the tert-butyl hydroperoxide is widely used in the study of cell damage caused by oxidative stress.

The experiment results are shown in Figs. 6A-6B, which show mean ± standard error of six experimental groups (n = 6). The statistical differences between damaging group (i.e. accepting damaging medicine, and no protective medicine) and testing medicine groups and the control group (Control, containing 0.5% of DMSO) were analyzed by Dunnett's multiple comparison test, which "*" represented $p < 0.05$; "**" represented $p < 0.01$; "***" represented $p < 0.001$; and "****" represented $p < 0.0001$.

As the experiment results shown in Figs. 6A-6B, the human retinal pigment epithelium cells in the existence of tBHP resulted in 30%-40% of cell death. However, after respectively adding Sample 2 (the concentrations were 6.25 μM, 12.5 μM, and 25 μM respectively) and Samples 8 (the concentrations were 8.75 μM, 17.5 μM, and 35 μM respectively), the human retinal pigment epithelium cell death could be significantly inhibited, and the cell viability were even better then the control.

Therefore, based on the results of Experimental example 6, the isoacteoside derivative of the embodiments of the present invention does have a protective effect on the oxidative stress damage of the human retinal pigment epithelium cells caused by tert-butyl hydroperoxide.

Given the above, the present invention provides an isoacteoside derivative, and the drug including the isoacteoside derivative has efficacy of inhibiting amyloid accumulation and preventing eye diseases.

Although the present invention has been described in considerable detail with reference to certain embodiments

thereof, other embodiments are possible. Therefore, the spirit and scope of the appended claims should not be limited to the description of the embodiments contained herein.

## Claims

**1.** An isoacteoside derivative, having a structure of formula (I):

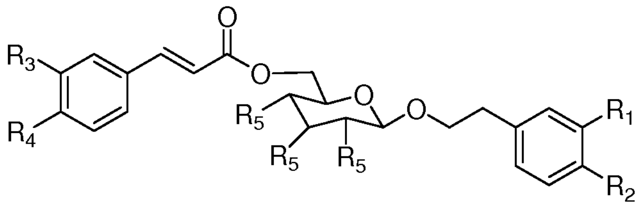
(I),

in formula (I), $R_1$ and $R_2$ being independently selected from hydrogen, halogen, a hydroxy group, or a hydrocarboxyl group, $R_3$ and $R_4$ being independently selected from a hydroxy group, a hydrocarboxyl group, or an acyloxy group, and $R_5$ being independently selected from a hydroxy group or an acyloxy group.

**2.** The isoacteoside derivative of claim 1, wherein when at least one of $R_1$ and $R_2$ is the hydrocarboxyl group, the at least one of $R_1$ and $R_2$ is independently selected from an alkoxy group, an alkenyloxy group, or an aryloxy group.

**3.** The isoacteoside derivative of claim 2, wherein when at least one of $R_1$ and $R_2$ is the alkoxy group, the at least one of $R_1$ and $R_2$ is a methoxy group.

**4.** The isoacteoside derivative of claim 2, wherein when at least one of $R_1$ and $R_2$ is the alkenyloxy group, the at least one of $R_1$ and $R_2$ is an allyloxy group.

**5.** The isoacteoside derivative of claim 2, wherein when at least one of $R_1$ and $R_2$ is the aryloxy group, the at least one of $R_1$ and $R_2$ is a benzyloxy group.

**6.** The isoacteoside derivative of claim 1, wherein when at least one of $R_3$ and $R_4$ is the hydrocarboxyl group, the at least one of $R_3$ and $R_4$ is independently selected from an alkenyloxy group or an aryloxy group.

**7.** The isoacteoside derivative of claim 6, wherein when at least one of $R_3$ and $R_4$ is the alkenyloxy group, the at least one of $R_3$ and $R_4$ is an allyloxy group.

**8.** The isoacteoside derivative of claim 6, wherein when at least one of $R_3$ and $R_4$ is the aryloxy group, the at least one of $R_3$ and $R_4$ is a benzyloxy group.

**9.** The isoacteoside derivative of claim 1, wherein when at least one of $R_3$ and $R_4$ is the acyloxy group, the at least one of $R_3$ and $R_4$ is an acetoxy group.

**10.** The isoacteoside derivative of claim 1, wherein $R_3$ and $R_4$ are the same substituent.

**11.** The isoacteoside derivative of claim 1, wherein when $R_5$ is the acyloxy group, $R_5$ is an acetoxy group.

**12.** The isoacteoside derivative of claim 1, wherein $R_5$ are the same substituent.

**13.** The isoacteoside derivative of claim 1, wherein the isoacteoside derivative is selected from following structures:

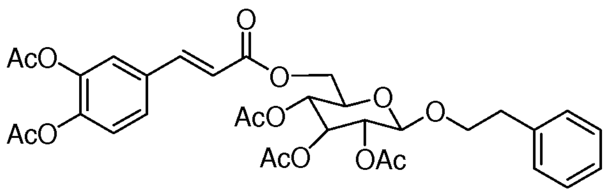
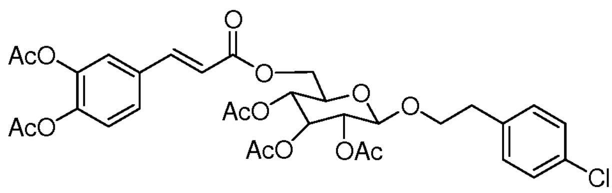
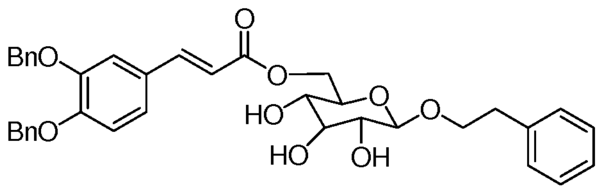
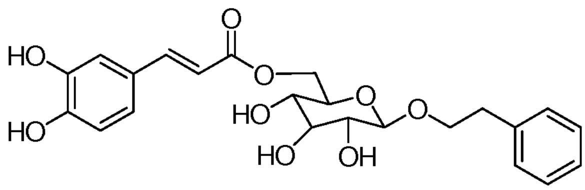
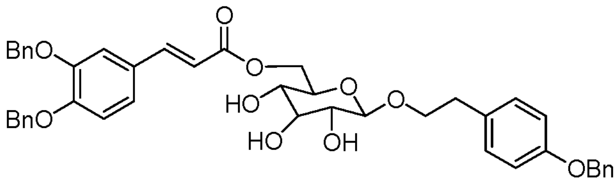
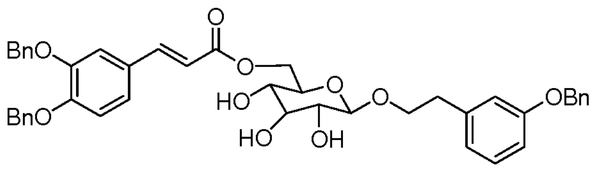
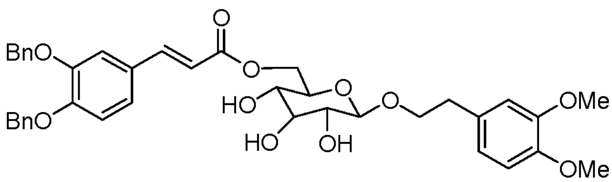
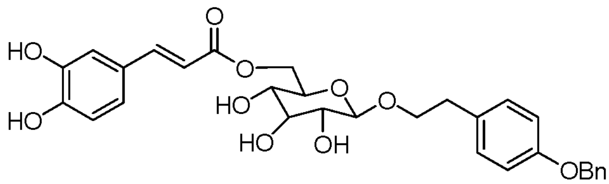
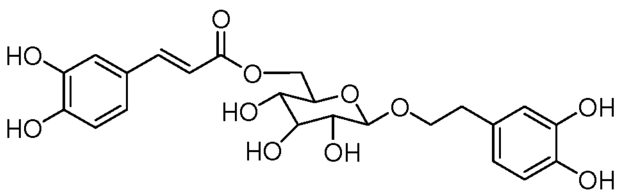

**14.** A method of preparing a medicine for treating or preventing an amyloid-related disease, comprising providing the isoacteoside derivative of claim 1 to prepare the medicine.

**15.** The method of claim 14, wherein the amyloid-related disease is a neurodegenerative disease.

**16.** The method of claim 14, wherein the amyloid-related disease is Alzheimer's disease, mild cognitive impairment, Lewy body dementia, Down's syndrome, hereditary cerebral hemorrhage with amyloidosis-Dutch type, Guam Parkinson-Dementia complex, progressive supranuclear palsy, multiple sclerosis, Creutzfeld Jacob disease, Parkinson's disease, frontotemporal dementia, Pick's disease, amyotrophic lateral sclerosis, inclusion-body myositis, adult-onset diabetes, senile cardiac amyloidosis, or endocrine tumor.

**17.** The method of claim 14, wherein the amyloid-related disease is an eye disease.

**18.** The method of claim 14, wherein the amyloid-related disease is neuronal degeneration, visual cortical defect, glaucoma, cataract, ocular amyloidosis, macular degeneration, optic nerve drusen, optic neuropathy, optic neuritis, or lattice corneal dystrophy.

**19.** The method of claim 14, wherein the amyloid is β-amyloid peptide.

**20.** A method for forming an isoacteoside derivative, comprising:

reacting a compound having a structure of formula (II) with β-D-glucose pentaacetate to form a compound having a structure of formula (III), wherein formula (II) is:

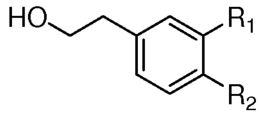

(II),

and formula (II) is:

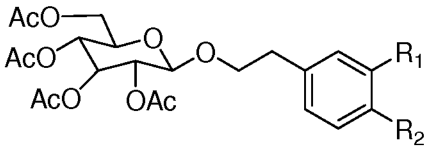

(III),

in formula (II) and formula (III), $R_1$ and $R_2$ being independently selected from hydrogen, chloride, or a methoxy group;

(1) reacting the compound having the structure of formula (III) with a mixture of palladium on carbon and methanol, after removing the palladium on carbon and purifying, mixing with potassium carbonate, allyl bromide, and acetone, and after refluxing, stirring in a potassium hydroxide-methanol solution to form a compound having a structure of formula (IV-1), wherein formula (IV-1) is:

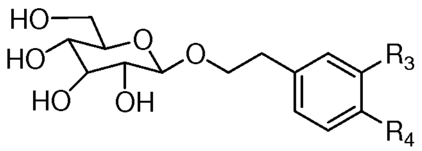

(IV-1),

in formula (IV-1), $R_3$ and $R_4$ being independently selected from hydrogen or an allyloxy group, (2) dissolving the compound having the structure of formula (III) in methanol and mixing with sodium methoxide to form the compound having the structure of formula (IV-1), wherein $R_3$ and $R_4$ are independently selected from hydrogen, chloride, a methoxy group, or a benzyloxy group, or (3) reacting the compound having the structure of formula (III) with acetyl chloride and methanol-dichloromethane to form a compound having a structure of formula (IV-2), wherein formula (IV-2) is:

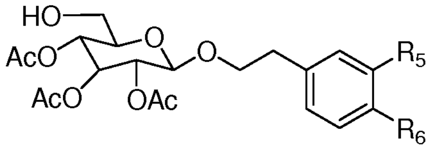

(IV-2),

in formula (IV-2), $R_5$ and $R_6$ being independently selected from hydrogen or chloride; and

reacting the compound having the structure of formula (IV-1) or the compound having the structure of formula (IV-2) with di-O-acetylferulic acid chloride, di-O-allylferulic acid chloride, or di-O-benzylferulic acid chloride in a solution of dichloromethane and pyridine to form a compound having a structure of any one of formulas (V-1)~(V-4), wherein formula (V-1) is:

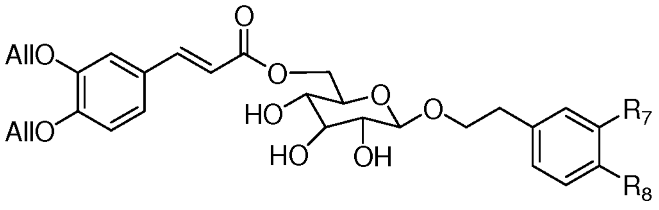

(V-1),

in formula (V-1), $R_7$ and $R_8$ being independently selected from hydrogen or an allyloxy group, formula (V-2) is:

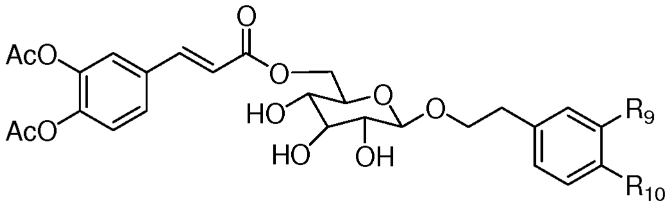

(V-2),

in formula (V-2), $R_9$ and $R_{10}$ being independently selected from hydrogen, a methoxy group, or a benzyloxy group, formula (V-3) is:

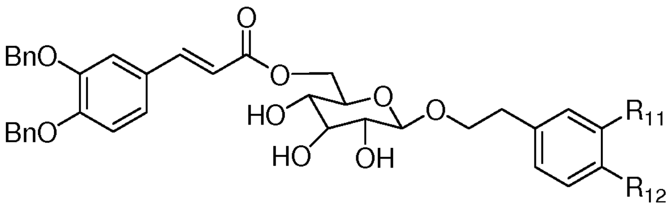

(V-3),

in formula (V-3), $R_{11}$ and $R_{12}$ being independently selected from hydrogen, a methoxy group, or a benzyloxy group, and formula (V-4) is:

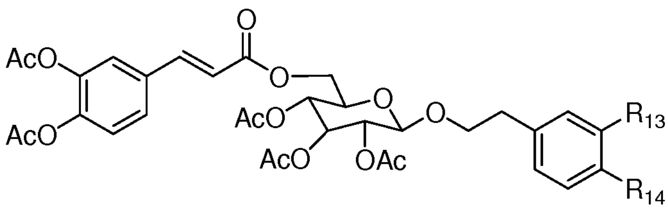

(V-4),

in formula (V-4), $R_{13}$ and $R_{14}$ being independently selected from hydrogen or chloride.

**21.** The method of claim 20, further comprising reacting the compound having the structure of formula (V-1) with copper(I) chloride and palladium dichloride in a mixture of methanol and water to form a compound having a structure of formula (VI-1), wherein formula (VI-1) is:

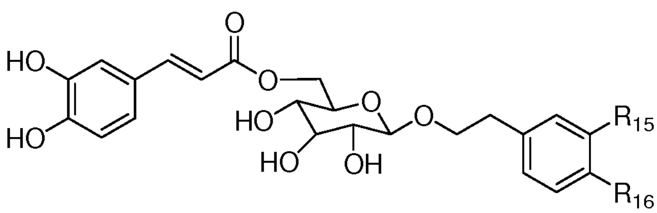

(VI-1),

in formula (VI-1), $R_{15}$ and $R_{16}$ being independently selected from hydrogen or a hydroxyl group.

**22.** The method of claim 20, further comprising reacting the compound having the structure of formula (V-2) with methylamine in methanol to form a compound having a structure of formula (VI-1), wherein formula (VI-1) is:

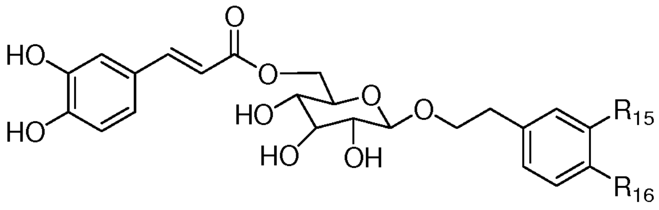

(VI-1),

in formula (VI-1), $R_{15}$ and $R_{16}$ being independently selected from hydrogen, chloride, a methoxy group, or a benzyloxy group.

**23.** The method of claim 20, further comprising reacting the compound having the structure of formula (V-4) with methylamine in methanol to form a compound having a structure of formula (VI-2), wherein formula (VI-2) is:

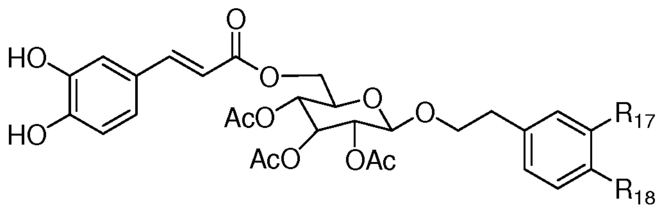

(VI-2),

in formula (VI-2), $R_{17}$ and $R_{18}$ being independently selected from hydrogen or chloride.

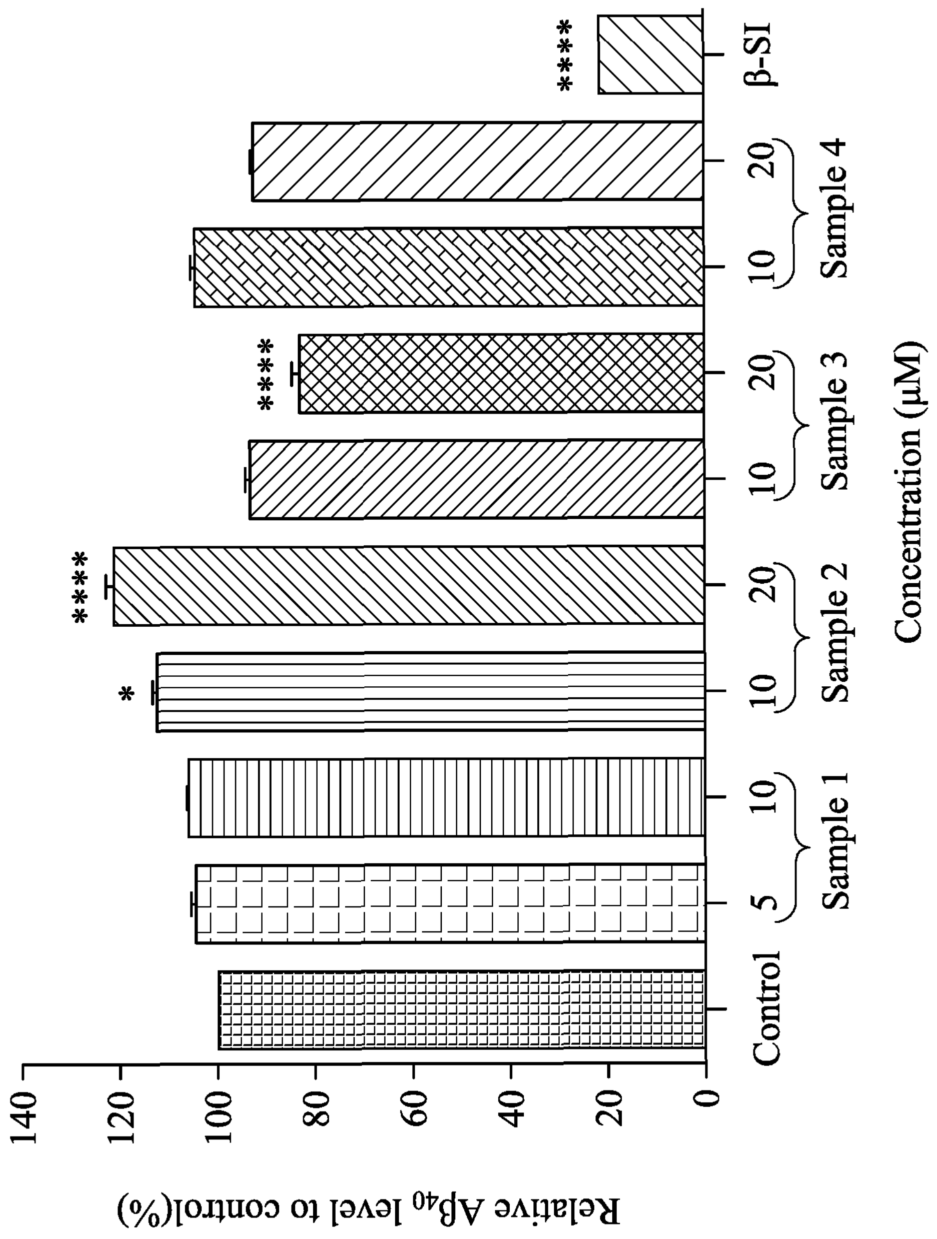
Relative Aβ40 level to control(%)
0
20
40
60
80
100
120
140
Control
5
10
Sample 1
10
20
Sample 2
10
20
Sample 3
10
20
Sample 4
β-SI
*
****
****
****
Concentration (μM)

Fig. 1A

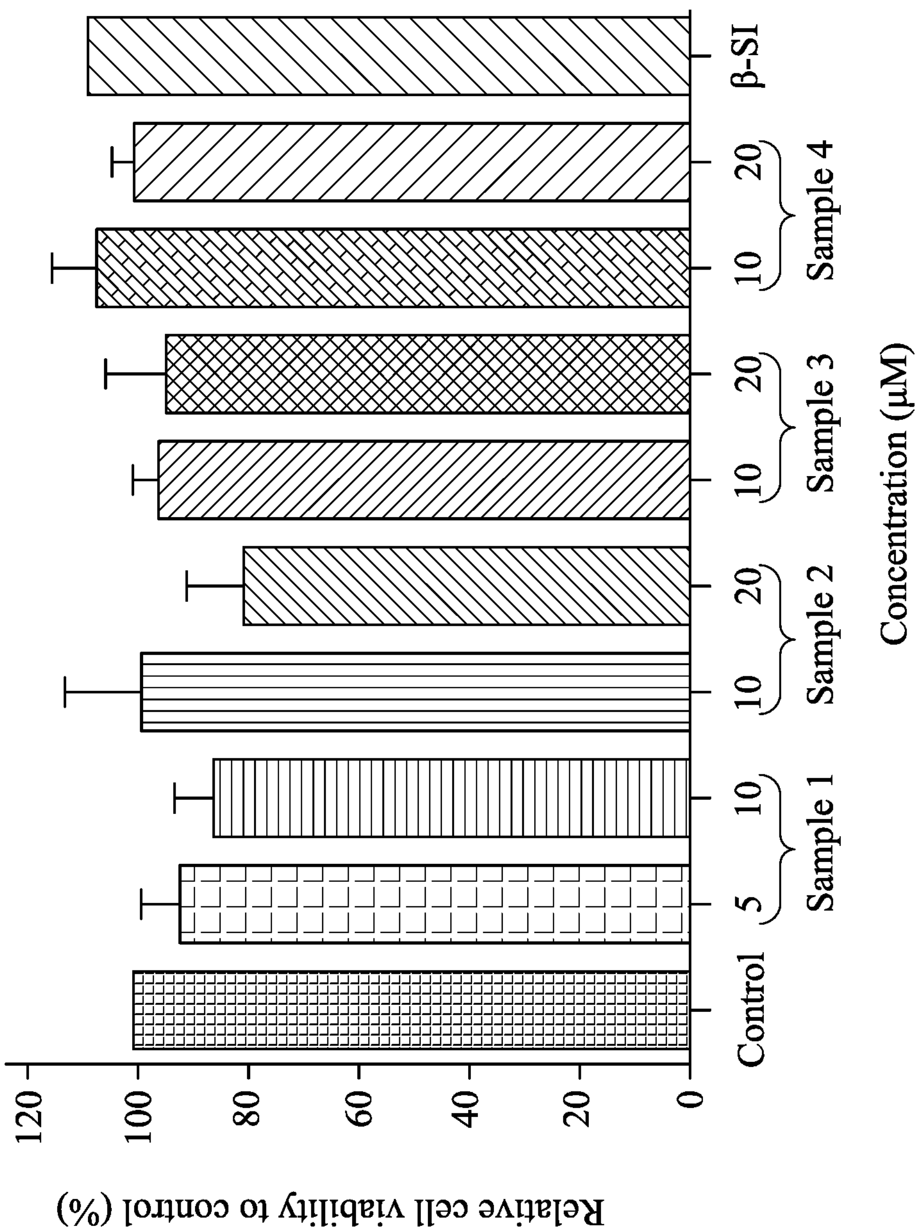
Relative cell viability to control (%)
0
20
40
60
80
100
120
Control
5
10
Sample 1
10
20
Sample 2
10
20
Sample 3
10
20
Sample 4
β-SI
Concentration (μM)

Fig. 1B

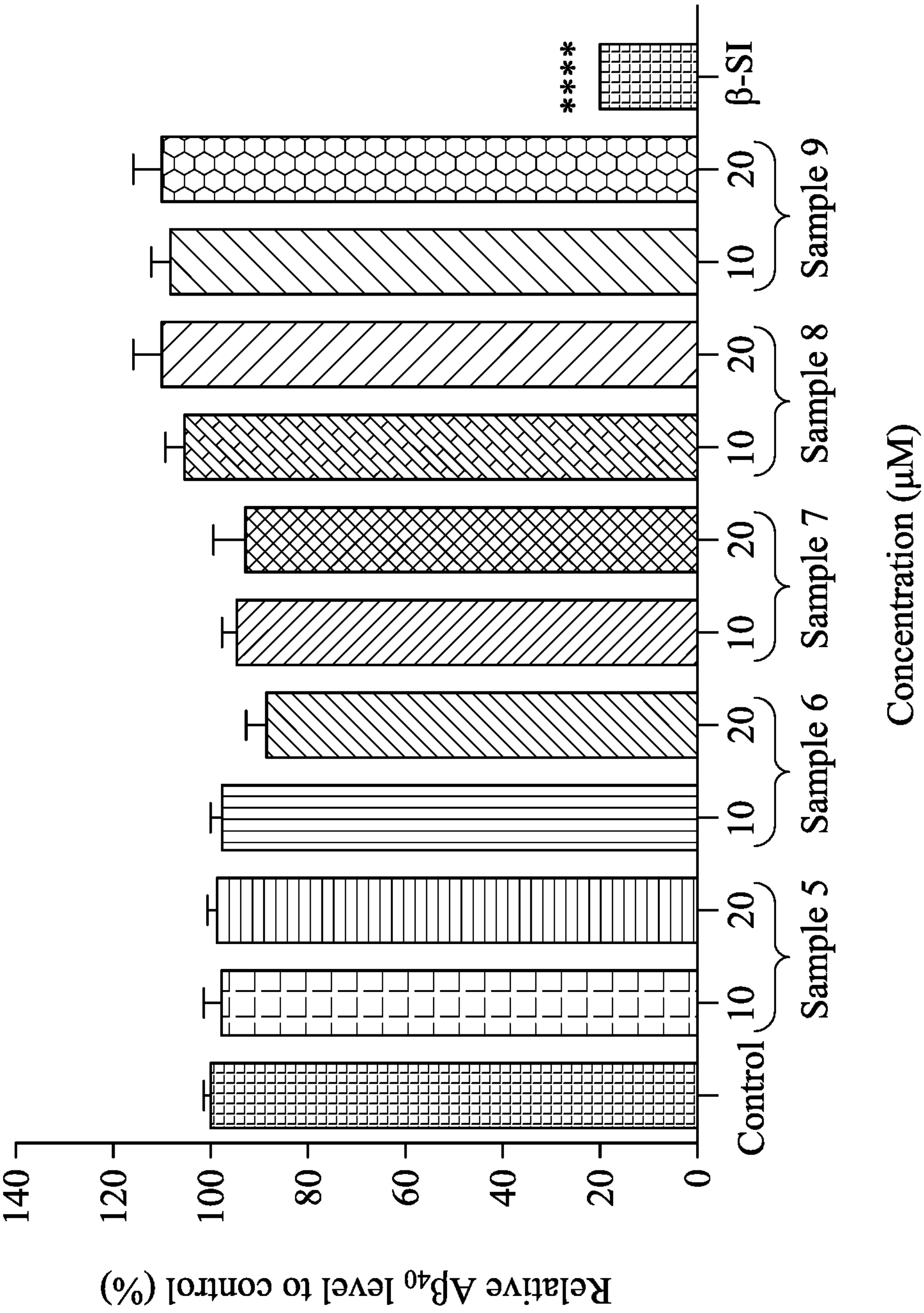
Relative Aβ40 level to control (%)
140
120
100
80
60
40
20
0
****
Control
10
20
Sample 5
10
20
Sample 6
10
20
Sample 7
10
20
Sample 8
10
20
Sample 9
β-SI
Concentration (μM)

Fig. 1C

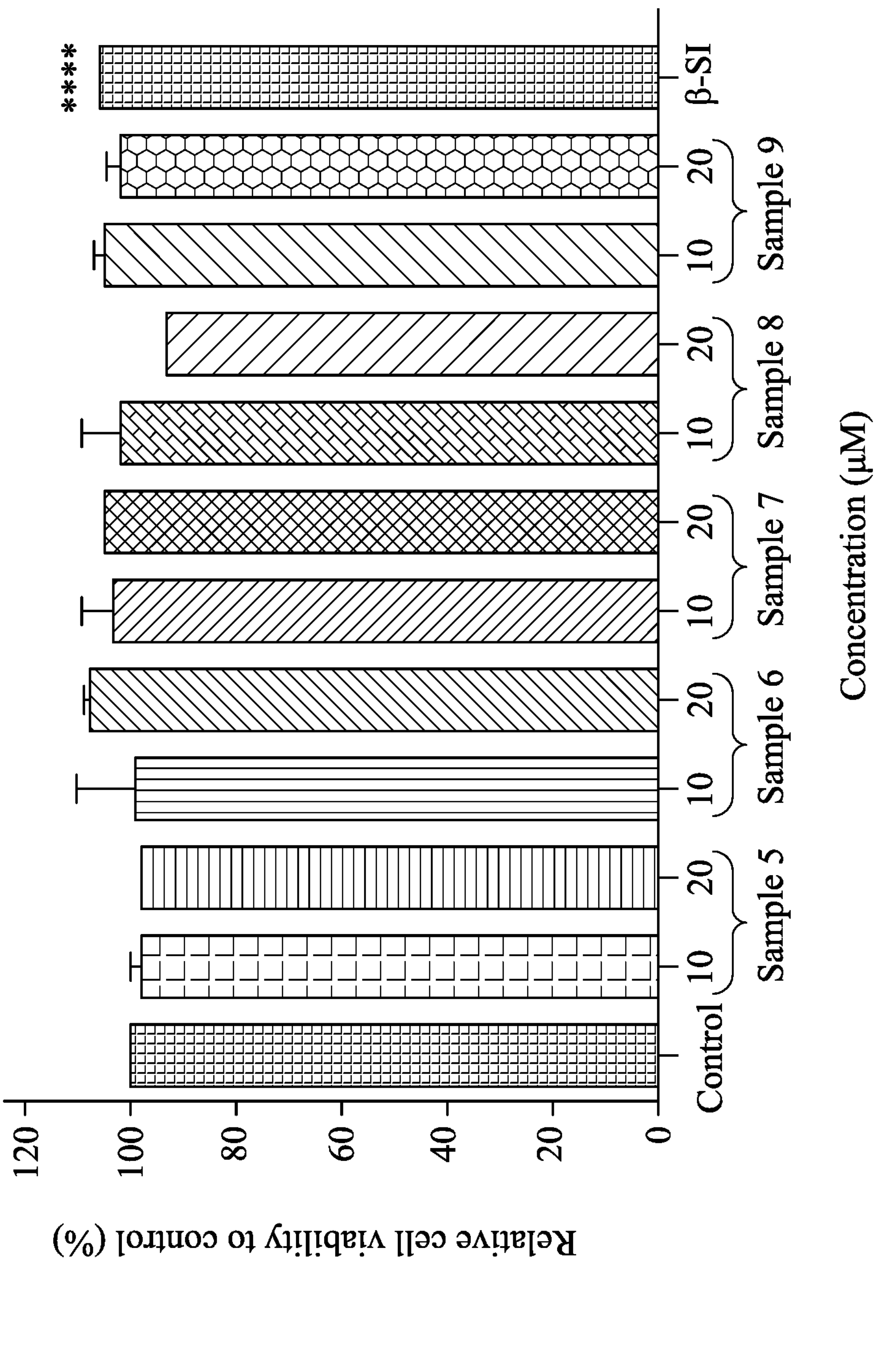
Relative cell viability to control (%)
0
20
40
60
80
100
120
Control
10
20
Sample 5
10
20
Sample 6
10
20
Sample 7
10
20
Sample 8
10
20
Sample 9
β-SI
****
Concentration (μM)

Fig. 1D

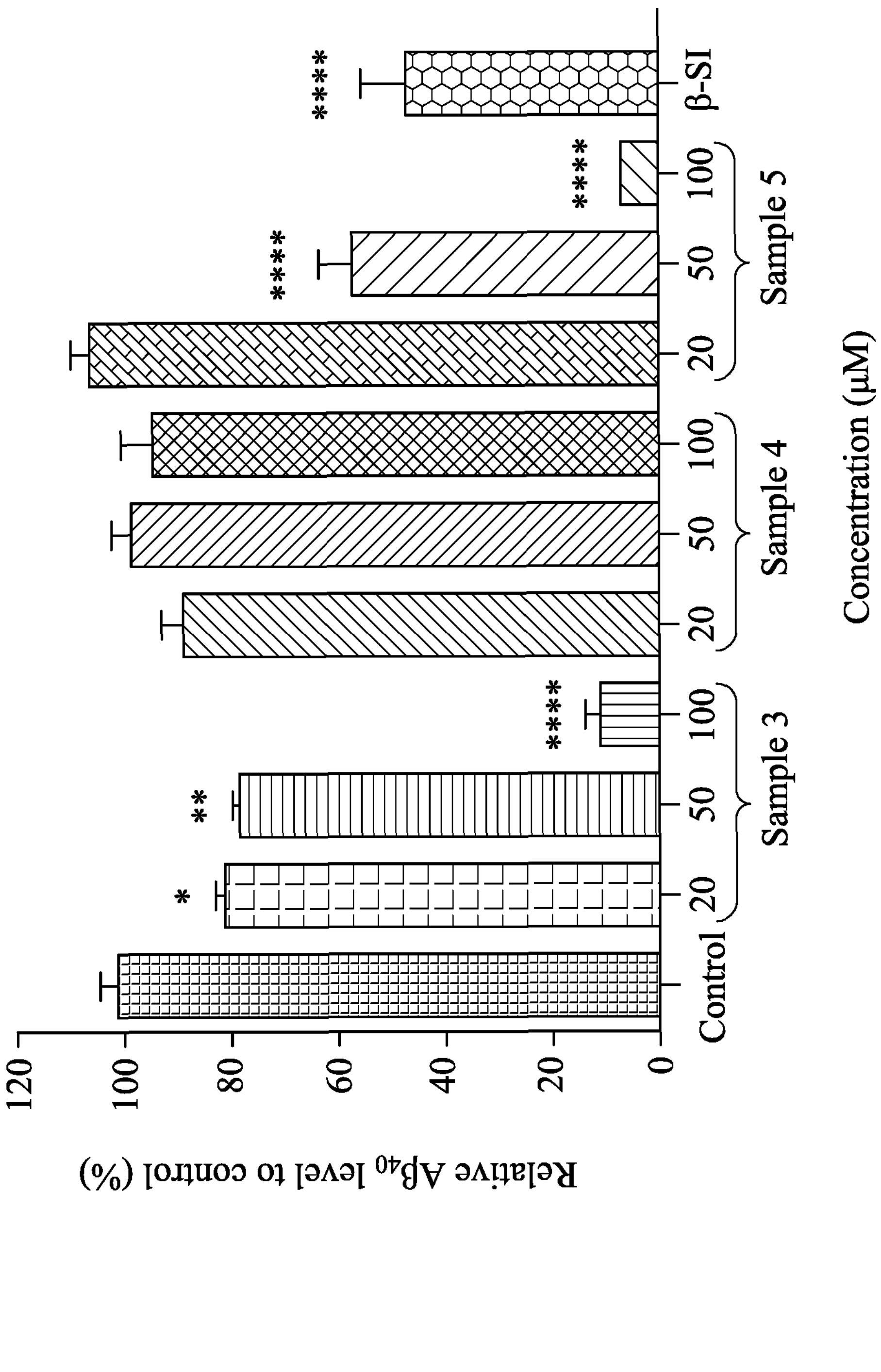
Relative Aβ40 level to control (%)
0
20
40
60
80
100
120
Control
20
50
100
Sample 3
20
50
100
Sample 4
20
50
100
Sample 5
β-SI
Concentration (μM)
*
**
****
****
****
****

Fig. 2A

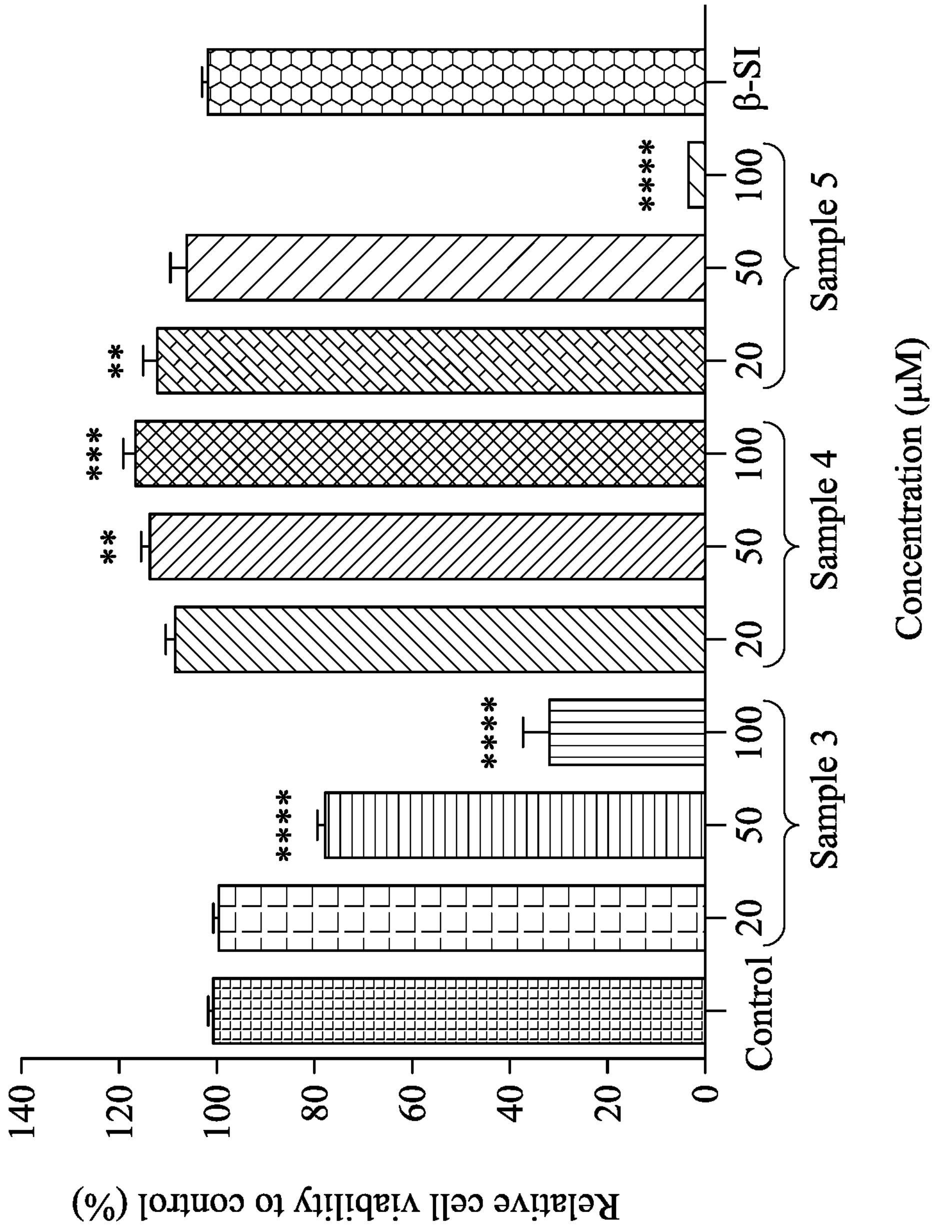
Relative cell viability to control (%)
0
20
40
60
80
100
120
140
Control
20
50
100
Sample 3
20
50
100
Sample 4
20
50
100
Sample 5
β-SI
Concentration (μM)
****
****
**
***
**
****

Fig. 2B

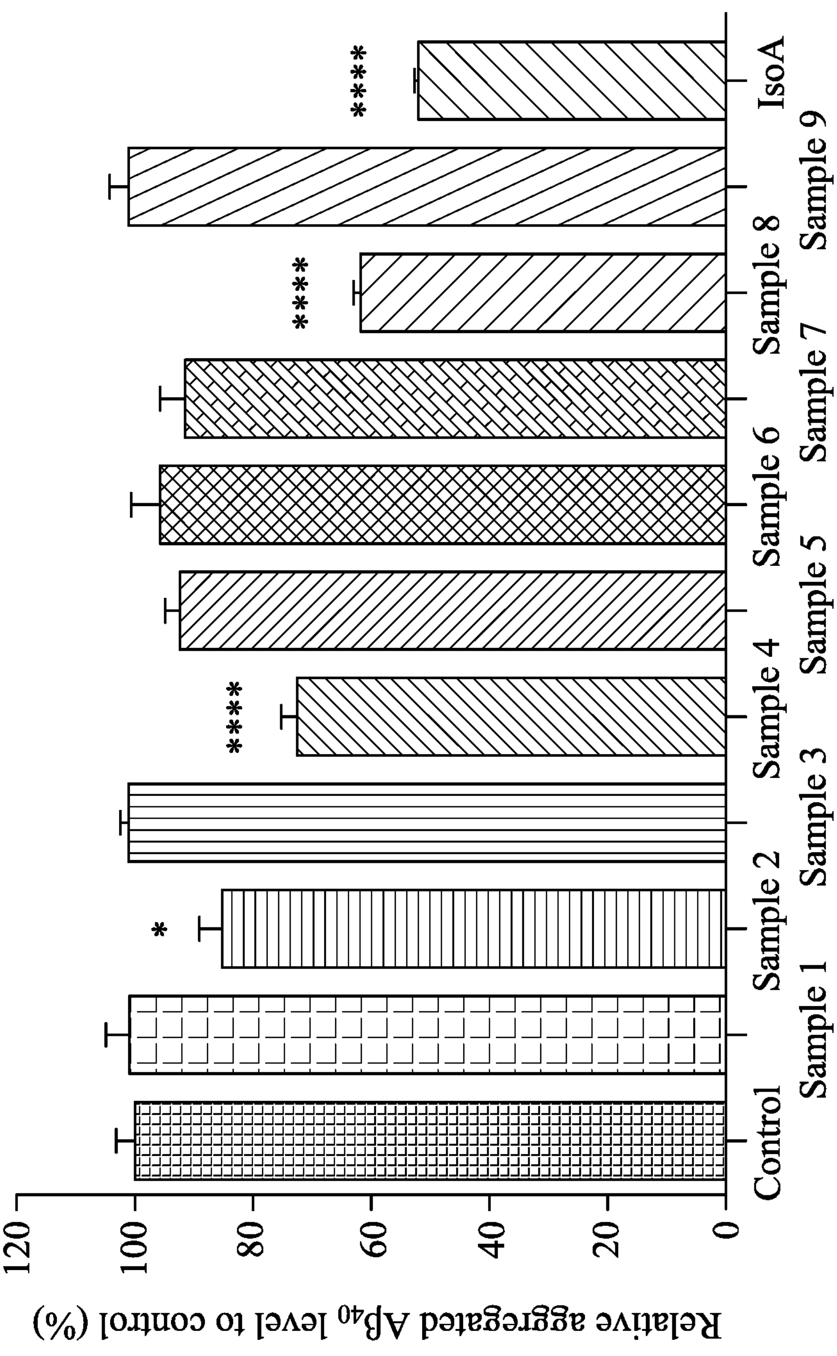
Relative aggregated Aβ40 level to control (%)
0
20
40
60
80
100
120
Control
Sample 1
Sample 2
Sample 3
Sample 4
Sample 5
Sample 6
Sample 7
Sample 8
Sample 9
IsoA
*
****
****
****

Fig. 3A

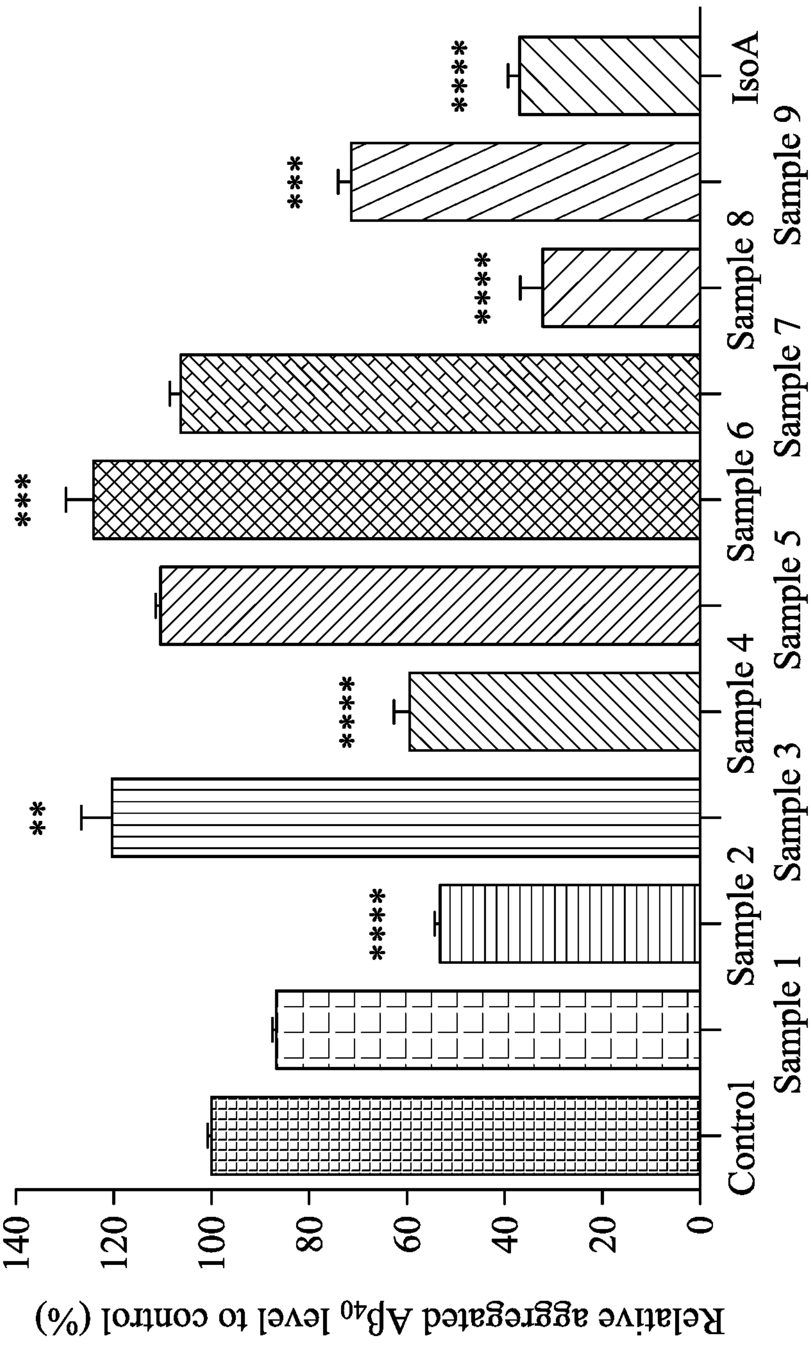
Relative aggregated Aβ40 level to control (%)
0
20
40
60
80
100
120
140
Control
Sample 1
Sample 2
Sample 3
Sample 4
Sample 5
Sample 6
Sample 7
Sample 8
Sample 9
IsoA
****
**
****
***
****
***
****

Fig. 3B

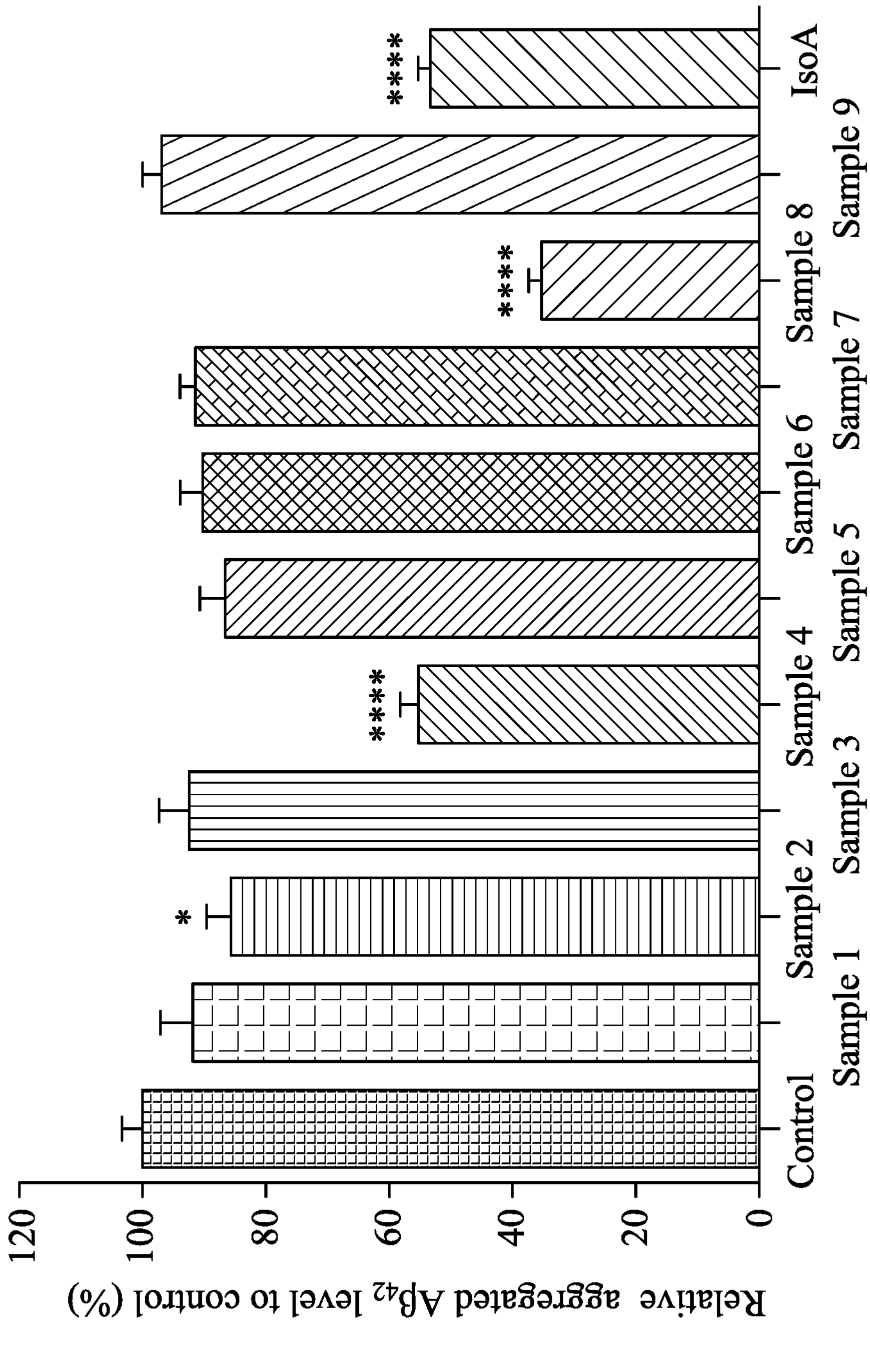
Relative aggregated Aβ42 level to control (%)
0
20
40
60
80
100
120
Control
Sample 1
Sample 2
Sample 3
Sample 4
Sample 5
Sample 6
Sample 7
Sample 8
Sample 9
IsoA
*
****
****
****

Fig. 4A

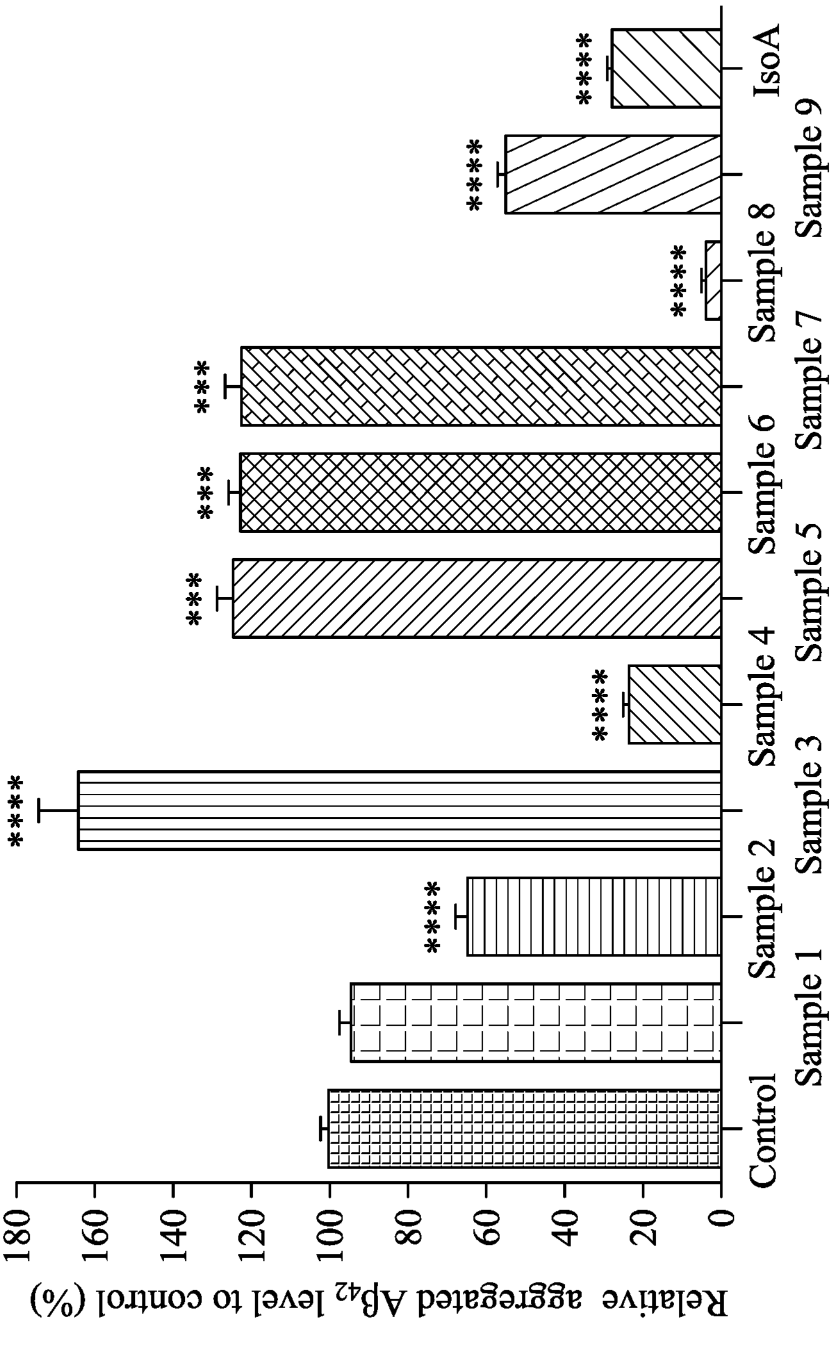
Relative aggregated Aβ42 level to control (%)
0
20
40
60
80
100
120
140
160
180
Control
Sample 1
Sample 2
Sample 3
Sample 4
Sample 5
Sample 6
Sample 7
Sample 8
Sample 9
IsoA
****
****
****
***
***
***
****
****
****

Fig. 4B

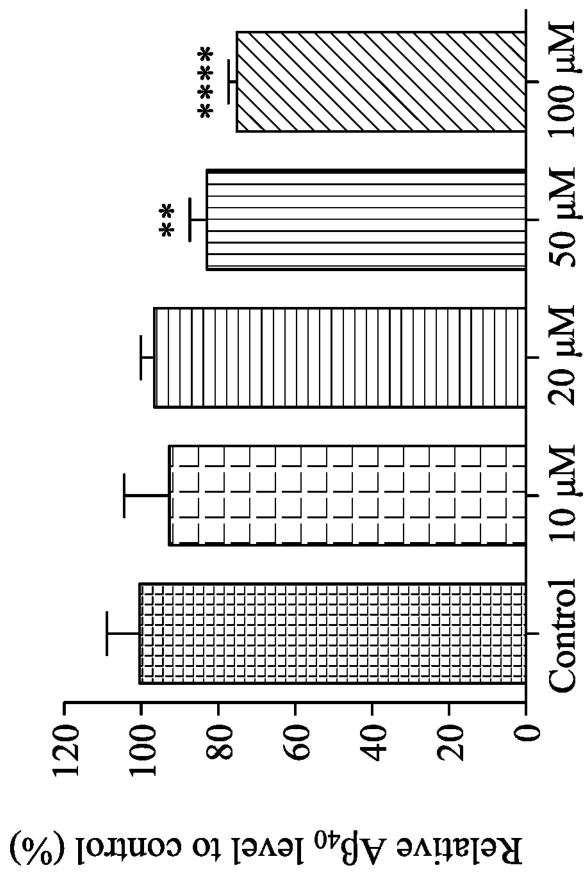
Relative Aβ40 level to control (%)
120
100
80
60
40
20
0
Control
10 µM
20 µM
50 µM
100 µM
**
****

Fig. 5

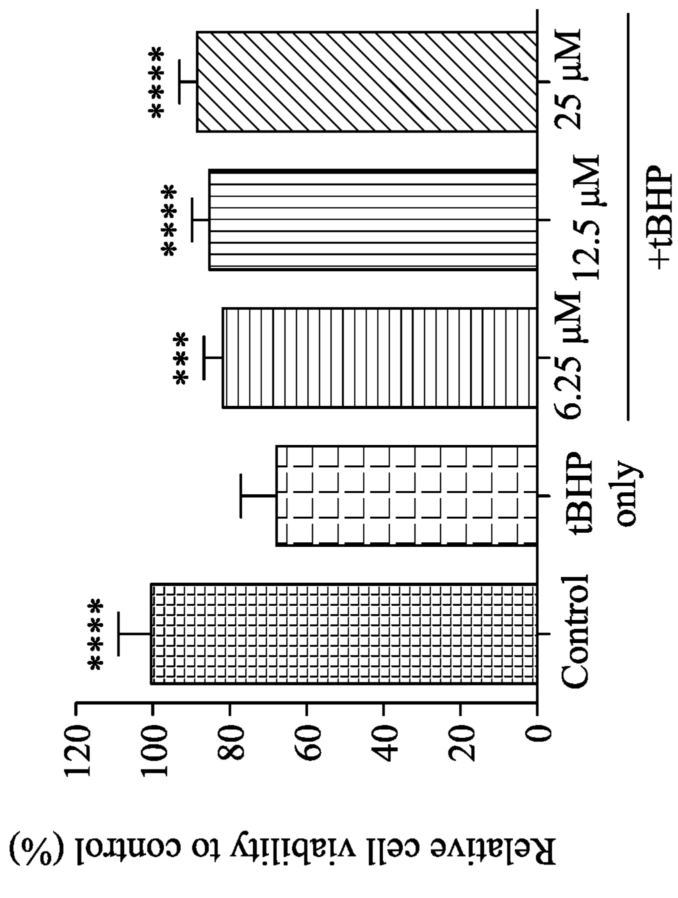
Relative cell viability to control (%)
0
20
40
60
80
100
120
Control
tBHP only
6.25 µM
12.5 µM
25 µM
+tBHP
****
***
****
****

Fig. 6A

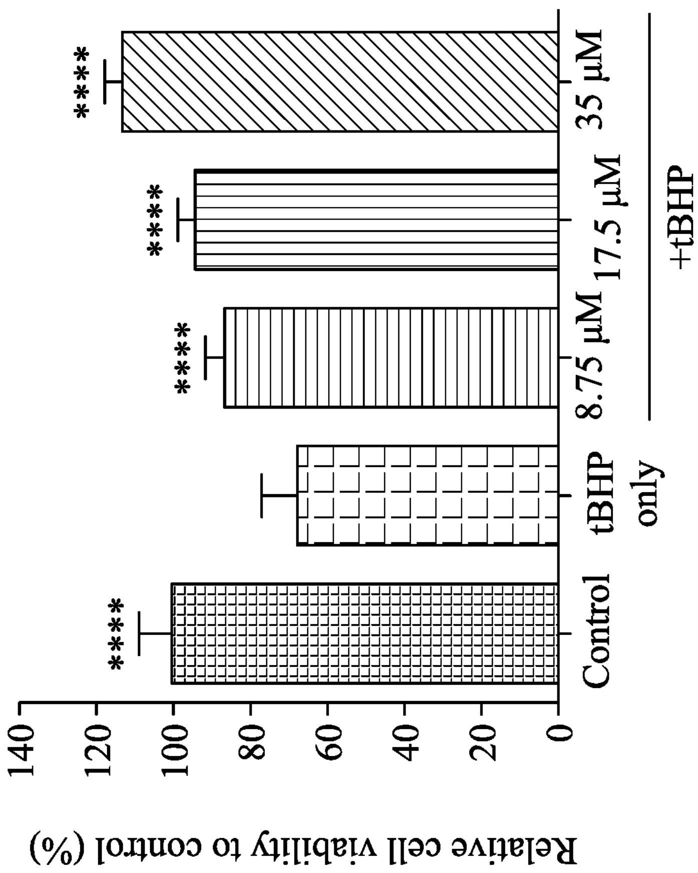
Relative cell viability to control (%)
140
120
100
80
60
40
20
0
****
****
****
****
Control
tBHP only
8.75 µM
17.5 µM
35 µM
+tBHP

Fig. 6B

# INTERNATIONAL SEARCH REPORT

International application No.
PCT/CN2015/076381

**A. CLASSIFICATION OF SUBJECT MATTER**

A61K 31/7032 (2006.01) i; A61K 31/7028 (2006.01) i; A61P 21/00 (2006.01) i; A61P 25/28 (2006.01) i;

According to International Patent Classification (IPC) or to both national classification and IPC

B. FIELDS SEARCHED

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

STN, CNABS, CNKI, CMPD, DWPI, SIPOABS, EMBASE: isoacteoside, amyloid peptides, Alzheimer's diseaes

C. DOCUMENTS CONSIDERED TO BE RELEVANT

| Category* | Citation of document, with indication, where appropriate, of the relevant passages | Relevant to claim No. |
|---|---|---|
| A | WO 2011157059 A1 (SINPHAR TIAN LI PHARMACEUTICAL CO., LTD. HANGZHOU) 22 December 2011 (22.12.2011) , see claim 1-22 | 1-23 |
| A | CN 103156867 A (SINPHAR TIAN-LI PHARM CO., LTD. HANGZHOU) 19 June 2013 (19.06.2013) , see claim 1 | 1-23 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 20 June 2015 | 13 July 2015 |

| Name and mailing address of the ISA | Authorized officer |
|---|---|
| State Intellectual Property Office of the P. R. China<br>No. 6, Xitucheng Road, Jimenqiao<br>Haidian District, Beijing 100088, China<br>Facsimile No. (86-10) 62019451 | YAO, Zhanghuan<br>Telephone No. (86-10) 62411131 |

Form PCT/ISA /210 (second sheet) (July 2009)

**INTERNATIONAL SEARCH REPORT**
Information on patent family members

International application No.
PCT/CN2015/076381

| Patent Documents referred in the Report | Publication Date | Patent Family | Publication Date |
|---|---|---|---|
| WO 2011157059 A1 | 22 December 2011 | TW 201200137 A | 01 January 2012 |
| | | US 2012009131 A1 | 12 January 2012 |
| | | EP 23997144 A1 | 21 December 2011 |
| | | AU 2011267724 A1 | 24 January 2013 |
| | | KR 20130095246 A | 27 August 2013 |
| | | CN 103037868 A | 10 April 2013 |
| | | CA 2802441 A1 | 22 December 2011 |
| | | JP 2013528627 A | 11 July 2013 |
| | | SG 186717 A1 | 2 February 2013 |
| | | EP 2397144 B1 | 31 December 2014 |
| CN 103156867 A | 19 June 2013 | WO 2013087042 A1 | 20 June 2013 |
| | | US 2015087606 A1 | 26 March 2015 |
| | | CA 2859227 A1 | 20 June 2013 |
| | | JP 2015500300 A | 05 January 2015 |
| | | AU 2012350437 A1 | 31 July 2014 |
| | | KR 20150002576 A | 07 January 2015 |
| | | EP 2792361 A4 | 13 May 2015 |
| | | TW 201325597 A | 01 July 2013 |
| | | EP 2792361 A1 | 22 October 2014 |

Form PCT/ISA /210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011157059 A1